(19) Europäisches Patentamt
European Patent Office
Office européen des brevets

(11) **EP 3 760 183 A1**

(12) **EUROPEAN PATENT APPLICATION**
published in accordance with Art. 153(4) EPC

(43) Date of publication:
**06.01.2021 Bulletin 2021/01**

(21) Application number: **20763976.6**

(22) Date of filing: **29.04.2020**

(51) Int Cl.:
$A61K\ 8/02^{(2006.01)}$     $A61K\ 8/27^{(2006.01)}$
$A61K\ 8/67^{(2006.01)}$     $A61K\ 8/64^{(2006.01)}$
$A61Q\ 19/00^{(2006.01)}$    $A61Q\ 19/10^{(2006.01)}$

(86) International application number:
**PCT/KR2020/005706**

(87) International publication number:
**WO 2020/226348 (12.11.2020 Gazette 2020/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.05.2019 KR 20190052504**

(71) Applicant: **Webiotree Co., Ltd.**
**Yeongdeungpo-gu**
**Seoul 07228 (KR)**

(72) Inventors:
• **KIM, Ho-Jun**
  **Seoul 04389 (KR)**
• **KIM, Youn-Jin**
  **Seoul 04389 (KR)**
• **KIM, Ki-Yeok**
  **Seoul 06625 (KR)**

(74) Representative: **Mitchell, Simon James**
**Urquhart-Dykes & Lord LLP**
**Euston House**
**24 Eversholt Street**
**London NW1 1AD (GB)**

(54) **NOVEL METAL LAYERED HYDROXIDE COMPOSITE AND PREPARATION METHOD THEREFOR**

(57)     The present invention relates to a metal layered hydroxide complex and a method of preparing the metal layered hydroxide complex.

FIG. 1

EP 3 760 183 A1

# Description

## Technical Field

The present invention relates to a novel metal layered hydroxide complex including an active ingredient and a peptide-based surface modifier and a method of preparing the same.

## Background Art

In order to improve the controlled releasability of a conventional metal layered hydroxide complex, etc. and adjust the molecular size thereof, surface treatment has been performed thereon using heat treatment, sonication or reconstruction. However, these surface treatment methods are problematic because decomposition of the active ingredient and unintentional release of the active ingredient may occur and also because a long processing time and a preparation facility therefor are required.

Moreover, with the goal of alleviating the problems with the above methods, which require a preparation facility, a polymer adsorption method or a dispersant treatment method may be applied, but has a long processing time and may use an organic solvent depending on the type of active ingredient, polymer or dispersant, and is thus limited in use. Furthermore, a process for post-treatment after main synthesis is performed, which is inefficient, and in particular, there is a disadvantage in that yield may be lowered during washing.

Accordingly, the surface modification of metal layered hydroxide is carried out through a delamination or exfoliation process by separating a nanosheet through swelling. To this end, however, an organic solvent such as toluene, formaldehyde, dichloromethane, etc. has to be used, which requires a post-treatment process such as washing and the like and causes biostability issues. Furthermore, complete separation of the nanosheet makes it impossible to stabilize the unstable active ingredient by introducing the same into a layered structure. Similarly, although a method using a ligand-binding functional group such as aminosilane, epoxysilane or vinylsilane is applied on the surface of the metal layer, it also causes the above problems due to the use of an organic solvent. Specifically, after washing and drying of the composite, the surface of particles undergoes a physical phenomenon of sharing water molecules between nanosheets due to hydrogen bonding and drying, and thus the particles may aggregate, so the particle size is ultimately larger. Moreover, upon surface treatment of the composite after washing and drying, a non-activated surface layer is formed due to the surface edging effect, and thus the interaction between particles is stabilized, and anion bonding or dispersion is very difficult, so overall surface modification efficiency may deteriorate, which is undesirable.

Conventionally, TEOS or TMOS, which is used as a surface modification agent as disclosed in Korean Patent Nos. 10-0439299 and 10-0841700, is composed mainly of silica, and thus exhibits controlled releasability but has high water content due to the properties of silica, undesirably causing problems related to the stability of the active ingredient. Also, when using lecithin, cellulose, xanthan gum, guar gum, methylcellulose, and hydroxypropyl methylcellulose disclosed in Korean Patent Application Publication No. 10-2006-0132409, the metal layered hydroxide and the modifier are merely provided in the form of being simply mixed, rather than being physically and chemically bound, so the particle size control and dispersion properties do not appear, which is undesirable.

As the conventional surface treatment methods described above, all of the high-temperature heat treatment or sonication method of changing the physical properties of the particle surface by etching the particle surface through treatment at about 100°C and sonication, the reconstruction method of inducing structural changes of particles through treatment at a high temperature of 200°C or more, the polymer or dispersant adsorption method of adsorbing the polymer to the surface of particles, the method of exfoliation of the metal layered hydroxide using the organic solvent, and the method of surface modification using the functional group are problematic because a preparation facility is required therefor or the preparation process is complicated and safety issues arise due to the use of the harmful solvent, and hence, alternative modification methods are required.

Therefore, the present invention is intended to provide a novel metal layered hydroxide complex, which does not have the aforementioned problems and is surface-modified.

(Patent Document 1) Korean Patent No. 10-0439299

(Patent Document 2) Korean Patent No. 10-0841700

(Patent Document 3) Korean Patent Application Publication No. 10-2006-0132409

**Disclosure**

**Technical Problem**

The present invention has been made keeping in the problems encountered in the related art, and an objective of the present invention is to provide a metal layered hydroxide complex, which includes an active ingredient and a surface modifier and has a reduced particle size and in which the active ingredient is stably contained and the controlled releasability thereof is significantly improved.

Another objective of the present invention is to provide a method of preparing a metal layered hydroxide complex, in which the metal layered hydroxide complex may be simply synthesized in a manner in which a metal layered hydroxide is bound to an active ingredient having a charge opposite thereto through electrostatic attraction or in which the internal structure of a metal layered hydroxide, that is, the crystallinity thereof, is adjusted, thereby making it possible to safely prepare the metal layered hydroxide complex without using harmful materials, achieving maximum synthesis efficiency and a short processing time and generating environmentally friendly and economical effects.

**Technical Solution**

The present invention provides a metal layered hydroxide complex including an active ingredient and a surface modifier, the active ingredient being at least one selected from the group consisting of ascorbic acid, biotin, pantothenic acid, cysteine, ferulic acid, glutamic acid, indole acetic acid, cinnamic acid, caffeic acid, thiamine, riboflavin, niacin, pantothenic acid, pyridoxine, folic acid, calciferol, tocopherol, tranexamic acid, salicylic acid, retinoic acid and arbutin, and the surface modifier including at least one selected from the group consisting of a peptide containing 2 to 8 amino acids and a peptide/fatty-acid conjugate including a peptide containing 2 to 8 amino acids and a fatty acid containing 10 to 16 carbon atoms.

In addition, the present invention provides a method of preparing the above metal layered hydroxide complex, including preparing a precursor solution by adding an acidic solution dropwise to a precursor of a metal layered hydroxide complex and dissolving the precursor and preparing a metal layered hydroxide complex by mixing the precursor solution with an alcohol, deionized water, an active ingredient, and a surface modifier.

In addition, the present invention provides a method of preparing the above metal layered hydroxide complex, including preparing a precursor solution by adding an acidic solution dropwise to a precursor of a metal layered hydroxide complex and dissolving the precursor, preparing a crystal seed containing an active ingredient by mixing the precursor solution with an alcohol, deionized water and an active ingredient, and preparing a metal layered hydroxide complex by mixing the prepared crystal seed with a surface modifier and performing an ion exchange reaction, in which the pH in the ion exchange reaction is 5 to 10.

**Advantageous Effects**

According to the present invention, there is provided a metal layered hydroxide complex including an active ingredient and a surface modifier, which is advantageous because of the reduced particle size, stable inclusion of the active ingredient, and vastly superior controlled releasability of the active ingredient.

Moreover, according to the present invention, the metal layered hydroxide complex can be simply synthesized in a manner in which a metal layered hydroxide is bound to an active ingredient having a charge opposite thereto through electrostatic attraction or in which the internal structure of a metal layered hydroxide, that is, the crystallinity thereof, is adjusted, thus achieving a short processing time and obviating a dedicated preparation facility, thereby generating environmentally friendly and economical effects.

In addition, according to the present invention, a method of preparing the metal layered hydroxide complex obviates post-treatment such as washing, etc. due to the use of an organic solvent such as toluene, formamide or dichloromethane, does not use harmful materials, and is therefore safe.

**Brief Description of Drawings**

FIG. 1 shows a process of forming a metal layered hydroxide complex using an outer ion-exchange process;

FIG. 2 shows a metal layered hydroxide complex formed using an outer ion-exchange process;

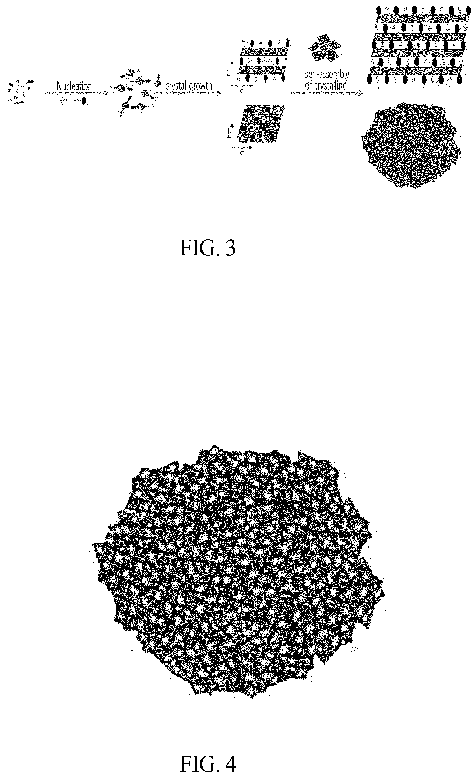

FIG. 3 shows a process of forming a metal layered hydroxide complex using a crossover interposition process;

FIG. 4 shows a metal layered hydroxide complex formed using a crossover interposition process;

FIGS. 5 to 10 are XRD graphs of the metal layered hydroxide complex;

FIGS. 11 to 20 are photographs showing the Tyndall effect for the metal layered hydroxide complex;

FIG. 21 shows the inner-outer structure of the metal layered hydroxide complex (in which the crystallinity thereof is adjusted at a ratio of SMA 0.005 : VitC 1) prepared using the outer ion-exchange process;

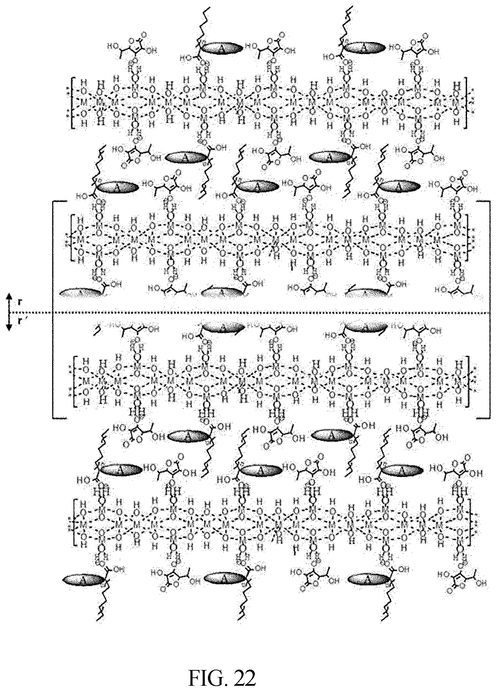

FIG. 22 shows the inner-outer structure of the metal layered hydroxide complex (in which the crystallinity thereof is adjusted at a ratio of SMA 0.5 : VitC 0.5) prepared using the crossover interposition process;

FIGS. 23 and 24 are graphs showing the results of evaluation of the particle size of the metal layered hydroxide complex;

FIGS. 25 and 26 are graphs showing the results of evaluation of zeta potential of the metal layered hydroxide complex;

FIGS. 27 and 28 are graphs showing the results of evaluation of the particle size of the metal layered hydroxide complex;

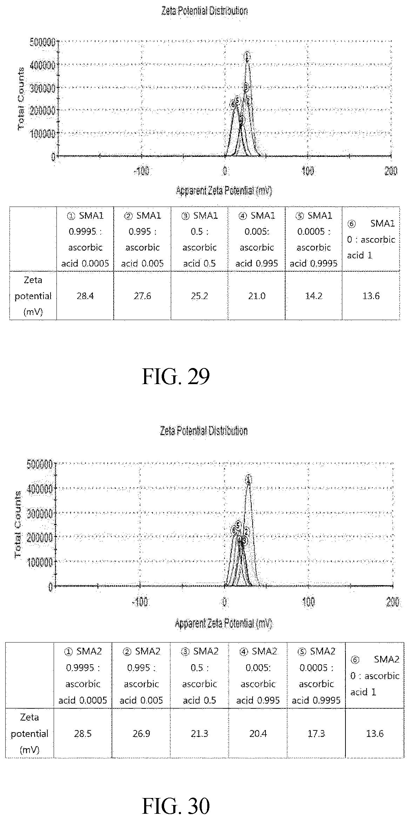

FIGS. 29 and 30 are graphs showing the results of evaluation of the zeta potential of the metal layered hydroxide complex; and

FIGS. 31 to 35 are graphs showing the results of evaluation of controlled releasability of the metal layered hydroxide complex.

**Best Mode**

The present invention pertains to a novel metal layered hydroxide complex including an active ingredient and a surface modifier and to a method of preparing the same. The metal layered hydroxide complex is applicable as a functional or pharmaceutical material such as an oral agent, cleanser, or shampoo, depending on the type of material inserted therein (e.g. a surfactant, amino acid, etc.), and is effective at maintaining the stability of the active ingredient and making the particle size small in colloidal form, thereby making it possible to increase the absorption capability thereof when used as a cosmetic and to exhibit superior controlled releasability of the active ingredient.

Specifically, the controlled releasability of the metal layered hydroxide complex according to the present invention enables the active ingredient to be released at a release rate of 65% or less within 72 hr, and preferably a release rate of 30 to 60% within 72 hr.

Moreover, the metal layered hydroxide complex of the present invention has a particle size (Z-average (d.nm)) of less than 1000 d.nm, and preferably 200 to 900 d.nm.

Hereinafter, a detailed description will be given of constructions of the present invention.

**<Novel metal layered hydroxide complex>**

In the present invention, a metal layered hydroxide complex may be a complex in which a metal and a hydroxide are bound to each other. Preferably, it is represented by Chemical Formula 1 below, but is not limited thereto.

[Chemical Formula 1]  $$[M^{2+}(O\{H\}_w)_{x'\times 0.5(1-w)}]A_{(2-x')} \cdot yH_2O$$

(in Chemical Formula 1,

$M^{2+}$ is $Mg^{2+}$, $Ca^{2+}$, $Co^{2+}$, $Cu^{2+}$, $Ni^{2+}$ or $Zn^{2+}$,

A is a negatively charged bioactive material,

x' is a number less than 2 but exceeding 1;

y is a number of 100 or less but exceeding 0; and

w is 0 or 1).

In the present invention, the active ingredient and the surface modifier are only so named so as to distinguish therebetween, and the surface modifier may serve as an active ingredient, and the active ingredient may also serve as a surface modifier.

In the present invention, the active ingredient may be at least one selected from the group consisting of ascorbic acid, biotin, pantothenic acid, cysteine, ferulic acid, glutamic acid, indole acetic acid, cinnamic acid, caffeic acid, thiamine, riboflavin, niacin, pantothenic acid, pyridoxine, folic acid, calciferol, tocopherol, tranexamic acid, salicylic acid, retinoic acid and arbutin.

In the present invention, the surface modifier may include at least one selected from the group consisting of a peptide containing 2 to 8 amino acids and a peptide/fatty-acid conjugate including a peptide containing 2 to 8 amino acids and a fatty acid containing 10 to 16 carbon atoms.

In the present invention, the abbreviations constituting each peptide generally correspond to the abbreviations typically used to express amino acids.

Specifically, in the present invention, the peptide containing 2 to 8 amino acids may be at least one selected from the group consisting of dipeptide-1 (YR), dipeptide-2 (VW), dipeptide-4 (FW), dipeptide-6 (KV), dipeptide-7 (KT), dipeptide-14 (AT), dipeptide (GH), acetyl dipeptide-1 (YR), acetyl dipeptide-1 cetyl ester (YR), nicotinoyl dipeptide-2 (VW), CP dipeptide (CP), VGE dipeptide (VE), CGE dipeptide (CE), EGE dipeptide (EE), TGE dipeptide (TE), LGE dipeptide (LE), EQ dipeptide (EQ), GR dipeptide (GR), HG dipeptide (HG), PE dipeptide (PE), DE dipeptide (DE), HQ dipeptide (HQ), RS dipeptide (RS), HP dipeptide (HP), carnosine (AH), tripeptide-1 (GHK), tripepdide-3 (GHR), tripeptide-4 (LGD), tripeptide-5 (KVK), tripeptide-6 (GXP), tripeptide-8 (HFR), tripeptide-10 (KDI), RGD peptide (RGD), AHK peptide (AHK), tripeptide-29 (GPX), tripeptide-54 (FTY), biotinoyl tripeptide-1 (GHK), thioctoyl tripeptide-1 (GHK), tripeptide (RFK), HGG peptide (HGG), peptide CK (RKR), tetrapeptide-1 (LPTV), tetrapeptide-2 (KDVY), tetrapeptide-3 (KGHK), tetrapeptide-5 (AHSH), tetrapeptide-7 (GQPR), tetrapeptide-9 (QDVH), tetrapeptide-11 (PPYL), tetrapeptide-15 (YPFF), tetrapeptide-21 (GEKG), tetrapeptide-26 (ELPS), acetyl tetrapeptide-2 (KDVY), acetyl tetrapeptide-3 (KGHK), acetyl tetrapeptide-5 (AHSH), acetyl tetrapeptide-9 (QDVH), acetyl tetrapeptide-11 (PPYL), acetyl tetrapeptide-15 (YPFF), pentapeptide-3 (GPRPA), pentapeptide-4 (KTTKS), pentapeptide-17 (KLAKK), pentapeptide-18 (YAGFL), thioctoyl pentapeptide-4 (KTTKS), hexapeptide-1 (ARHLFW), hexapeptide-2 (FWFKPV), hexapeptide-3 (EEMQRR), hexapeptide-4 (FGHXAF), hexapeptide-5 (FGVXAF), hexapeptide-6 (VEPIPY), hexapeptide-9 (GPQGPQ), hexapeptide-11 (FVAPFP), hexapeptide-12 (VGVAPG), acetyl hexapeptide-1 (ARHLFW), acetyl hexapeptide-3 (EEMQRR), acetyl hexapeptide-6 (VEPIPY), heptapeptide-1 (EDDDWDF), heptapeptide-7 (MGRNIRN), cysteine peptide (RFAACAA), lysine peptide (RFAAKAA), selank (TKPRPGP), octapeptide-2 (TAEEHEVM), octapeptide-3 (EEMQRRAD), octapeptide-4 (YGGFLGHK) and acetyl octapeptide-3 (EEMQRRAD).

In the present invention, the peptide/fatty-acid conjugate may be at least one selected from the group consisting of palmitoyl dipeptide-6 (KV), palmitoyl dipeptide-7 (KT), palmitoyl carnosine (AH), azelaoyl tripeptide-1 (GHK), palmitoyl-tripeptide-3 (GHR), myristoyl tripeptide-5 (KVK), palmitoyl tripeptide-1 (GHK), palmitoyl tripeptide-5 (KVK), palmitoyl tripeptide (RFK), myristoyl tripeptide-1 (GHK), palmitoyl tripeptide-4 (LGD), palmitoyl tripeptide-8 (HFR), palmitoyl tetrapeptide-7 (GQPR), myristoyl pentapeptide-17 (KLAKK), palmitoyl pentapeptide-4 (KTTKS), palmitoyl pentapeptide-17 (KLAKK), myristoyl hexapeptide-12 (VGVAPG) and palmitoyl hexapeptide-12 (VGVAPG).

In the present invention, the novel metal layered hydroxide complex may be applied to cosmetics, cleansers, pharmaceuticals, etc., depending on the types of active ingredient and surface modifier. In particular, the functionality of the prepared material may vary depending on the properties of the anions used as the active ingredient and/or the surface modifier. Specifically, a product prepared using ascorbic acid as the active ingredient and the surface modifier may be utilized as a cosmetic having increased absorption capability when applied to the skin, or a product prepared using ascorbic acid as the active ingredient and a surfactant as the surface modifier may function as a cleanser having antioxidant efficacy.

Also, the metal layered hydroxide complex of the present invention may be used as a cosmetic or pharmaceutical composition. A pharmaceutical composition including the metal layered hydroxide complex of the present invention may further include an appropriate carrier, excipient or diluent in accordance with typical processes. Examples of the carrier, excipient and diluent that may be included in the pharmaceutical composition containing the compound may include, but are not limited to, lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil.

Also, the pharmaceutical composition of the present invention may be formulated into any one dosage form selected from the group consisting of powders, pills, granules, capsules, suspensions, solutions for internal use, emulsions, syrups, sterilized aqueous solutions, non-aqueous solutions, lyophilized agents and suppositories, in accordance with typical processes. When the pharmaceutical composition of the present invention is formulated, a typical diluent or excipient, such as a filler, an extender, a binder, a wetting agent, a disintegrant, a surfactant, and the like, may be used.

A solid formulation for oral administration may include tablets, pills, powders, granules, capsules, and the like, and such a solid formulation may be prepared by mixing the above compound with at least one excipient, for example, starch, calcium carbonate, sucrose, lactose, gelatin, or the like. In addition to the simple excipient, lubricants such as magnesium stearate, talc and the like may be used. An oral liquid formulation may include suspensions, solutions for internal use, emulsions, syrups, and the like, and may also include not only simple diluents, such as water or liquid paraffin, but also various excipients, for example, wetting agents, sweeteners, fragrances, preservatives and the like. A formulation for parenteral administration may include sterilized aqueous solutions, non-aqueous solvents, suspensions, emulsions, lyophilized formulations and suppositories. As non-aqueous solvents or suspension agents, propylene glycol, polyethylene glycol, vegetable oil such as olive oil, injectable esters such as ethyl oleate and the like may be used. As the base of a suppository, Witepsol, Macrogol, Tween 60, cacao butter, laurin fat, glycerogelatin and the like may be used.

The pharmaceutical composition including the metal layered hydroxide complex of the present invention may be administered orally or parenterally (for example, intravenously, subcutaneously, intraperitoneally or topically) depending on the purpose of use, and the amount of the pharmaceutical composition according to the present invention, when administered, may vary depending on the patient's health status, weight, age, gender, diet, excretion rate, disease severity, drug form, administration time, administration route and administration period, and may be appropriately determined by those skilled in the art. The scope of the present invention is not limited thereby in any way.

The cosmetic or pharmaceutical composition of the present invention may specifically be a formulation for external application to the skin, and more specifically, the formulation for external application to the skin may include any one selected from the group consisting of a solution, suspension, emulsion, paste, gel, cream, lotion, powder, soap, surfactant-containing cleanser, oil, powder foundation, emulsion foundation, wax foundation and spray, but is not limited thereto, so long as it is a formulation used for application to the skin.

**<Method of preparing metal layered hydroxide complex>**

The metal layered hydroxide complex of the present invention may be prepared using an outer ion-exchange process or a crossover interposition process, but the present invention is not limited thereto. The method of preparing the metal layered hydroxide according to the present invention makes it possible to control the particle size and exhibit superior dispersibility. The above method is a method of surface modification by growing the particles to the maximum size by allowing the maximum aggregation growth time for a crystal seed produced by nucleation after the start of synthesis, and is preferable from the following point of view: since the binding force between the particles in the aqueous solution before washing is weaker than after washing and drying (because different counter anions or metal salts are present in the aqueous solution), the particle size is small and the surface area between particles is high, so surface treatment is very useful. Surface modification of the particles is performed using the properties between anionic materials containing functional groups (functional groups: hydroxyl group (-OH), carbonyl group of ketone (-CO-), formyl group of aldehyde (-CHO), carboxyl group of organic acid (-COOH), amino group of amine ($-NH_2$), sulfate, phosphate, etc.) having electrostatic attraction depending on the pKa, the activity of which is very high in an aqueous solution.

In the present invention, specific examples of the precursor of the metal layered hydroxide complex may include $ZnO$, $ZnSO_4$, $ZnCl_2$, $ZnCO_3$, $Zn(NO_3)\text{-}6H_2O$, $Zn(CH_3COO)_2$, $CaO$, $CaCl_2$, $Ca(NO_3)_2 6H_2O$, $CaSO_4$, $CaCO_3$, $Ca(OH)_2$, $MgO$, $MgCl_2$, $MgSO_4$, $Mg(NO_3)_2 6H_2O$, $CuO$, $Cu(NO_3)_2 6H_2O$, $CuCl_2$, $CuSO_4$, $Co(NO_3)_2$, $CoCO_3$, $CoCl_2$, $Co(OH)_2$, $CoSO_4$, $Co(CH_3COO)_2$, $NiO$, $NiCl_2$, $Ni(NO_3)_2$, $NiSO_4$, $NiCO_3$, $Ni(OH)_2$, $FeO$, $FeCl_3$, $Fe(NO_3)_3$, $FeSO_4$, $FeCO_3$, and $Fe(OH)_2$. Preferably, $ZnO$, $ZnSO_4$, $ZnCl_2$, $ZnCO_3$, $Zn(NO_3)_2 6H_2O$, or $Zn(CH_3COO)_2$ is used.

Specifically, the outer ion-exchange process may include preparing a precursor solution by adding an acidic solution dropwise to a precursor of a metal layered hydroxide complex and dissolving the precursor therein, preparing a crystal seed containing an active ingredient by mixing the precursor solution with an alcohol, deionized water, an active ingredient, etc., and preparing a metal layered hydroxide complex by mixing the prepared crystal seed with a surface modifier and performing an ion exchange reaction. More specifically, the outer ion-exchange process is a synthesis method in which the aggregation growth time of the seed particles bound to the active ingredient is maximized to thus increase the size of crystals in a predetermined period of time, followed by ion exchange on the surface of the particles using a surface modifier before washing. When the pH in the ion exchange reaction ranges from 5 to 10, it is possible to minimize the release of the active ingredient due to the pH change by maintaining the existing pH condition for synthesis of metal layered hydroxide, and moreover, collapse of the metal layered hydroxide structure may be prevented, thus increasing the controlled releasability of the active ingredient.

As for the peptide used in the ion exchange reaction, a peptide having a PI of 5 to 10, which satisfies the pH condition ranging from 5 to 10, is preferable. In the present invention, PI (isoelectric point) refers to the electrostatic properties of a peptide, and means the average value represented by the electrostatic properties of the dipolar structure of pKa of the peptide. It is closely related to the pKa of the peptide; for example, the PI of an amino acid containing one amine group and one carboxyl group may be represented by the equation $(pK_{a1} + pK_{a2})/2 = PI$, and the PI value of the peptide may be easily derived by a commonly used PI calculation method. The peptide having a PI value at a pH of 5 to 10 has a bonding active group suitable for hydrogen bonding, carboxylate bonding, or the like, and thus may exhibit superior electrostatic attraction during ion exchange.

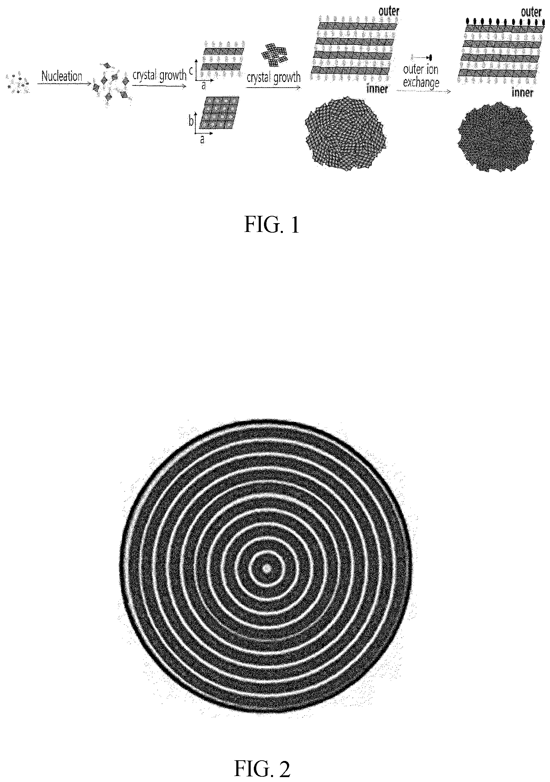

When the metal layered hydroxide complex of the present invention is prepared, the active ingredient and the surface modifier may be mixed at a molar ratio of 1:0.0005 to 0.0005:1. Given the above range, controlled releasability of the active ingredient may be effectively adjusted, and moreover, the particle size of the metal layered hydroxide may be decreased, and the unnecessary use of coating agent may be reduced. More specifically, in the ion exchange reaction, surface modification is carried out using anion exchange capacity. The outer ion-exchange process enables the formation of a structure configured such that the outermost surface of the metal layered hydroxide complex is surrounded by the surface modifier layer (FIGS. 1 and 2).

In addition, the crossover interposition process may include preparing a precursor solution by adding an acidic solution dropwise to a precursor of a metal layered hydroxide complex and dissolving the precursor therein and preparing a metal layered hydroxide complex by mixing the precursor solution with an alcohol, deionized water, an active ingredient, and a surface modifier. When the metal layered hydroxide complex of the present invention is prepared, the active ingredient and the surface modifier may be mixed at a molar ratio of 0.9995:0.0005 to 0.0005:0.9995. The crossover interposition process enables the formation of a structure configured such that the active ingredient and the surface modifier are provided in the form of a mixture both inside and on the outermost surface of the metal layered hydroxide complex (FIG. 2).

**Mode for Invention**

Specific preparation methods according to the present invention are described below. However, the present invention is not limited to the following examples.

**Preparation Examples**

**Preparation Example 1: Preparation of metal layered hydroxide complex using outer ion-exchange process**

As basic conditions for the experiment, all processes were performed at room temperature (20°C) and in a nitrogen atmosphere. Moreover, 95% ethanol and deionized water were used, and ascorbic acid (AA) and SMA (surface modifier), having a purity of 95% or more (water content of 5% or less), were used. A 3.2 M NaOH aqueous solution was prepared using deionized water.

SMA1 and SMA2, which are the surface modifiers used in the present invention, were pentapeptide-4 and palmitoyl dipeptide-7, respectively.

The experiment was carried out as follows in the order of synthesis, washing and drying.

2.5 equivalent weights of ZnO were placed in a main tank, after which a concentrated HCl solution in an auxiliary tank 1 was slowly added dropwise to the main tank so that the pH was titrated to 0.5-1, followed by dissolution with stirring at 700 rpm for about 30 min in a nitrogen atmosphere.

Thereafter, 1 equivalent weight of AA in an auxiliary tank 2, deionized water, and ethanol were placed in the main tank (the total volume ratio of the solvent that was added relative to Zn was 20) and were then dissolved with stirring at 300 rpm for about 5 min. A 3.2 M NaOH solution in an auxiliary tank 3 was added to the main tank while the stirring rate of the main tank was maintained, so the pH in the main tank was titrated in the range of 6.5 to 7.5, after which a precipitation reaction was induced in the main tank with stirring for 3 hr. Here, the pH was maintained at 6.5-7.5.

Next, the precipitate was washed. Specifically, in order to remove the unreacted salt in the above solution and the ionic material, impurities were removed using a centrifuge. The centrifugation process was performed three times for 5 min each at 8000 rpm, and the precipitate and the solution were separated from each other as follows. The synthesized precipitate solution was centrifuged at 8000 rpm for 5 min. After separation of the solution and the precipitate, the precipitate was uniformly diluted with ethanol and deionized water at a volume ratio of 1:1 and then centrifuged at 8000 rpm for 5 min. This procedure was repeated three times. In the final centrifugation washing process, the precipitate was diluted using ethanol alone and then centrifuged at 9000 rpm for 10 min, thereby yielding a precipitate.

Next, surface modification was performed. Specifically, the precipitate separated after washing was diluted using ethanol and deionized water at a volume ratio of 1 : 9 to 9 : 1 in a nitrogen atmosphere and then stirred at 700 rpm for 30 min. The dispersed precipitate solution was very slowly added with a solution of SMA relative to AA at a molar ratio of 1 : 0.05, 1 : 0.025, 1 : 0.005, 1 : 0.0025, and 1 : 0.0005 dissolved in ethanol (50%), and then stirred for about 1 to 3 hr while the pH thereof was maintained at pH 6-8 using a weak alkaline solution. After completion of the reaction, the precipitate was centrifuged at 8000 rpm. After separation of the solution and the precipitate from each other, the precipitate was uniformly diluted with ethanol and deionized water at a ratio of 1:1 and then centrifuged at 8000 rpm for 5 min. This procedure was repeated three times. In the final centrifugation washing process, the precipitate was diluted using ethanol alone and then centrifuged at 9000 rpm for 10 min, thus yielding a precipitate. The washed precipitate solution was spray dried with stirring at 600 rpm, thereby obtaining a powder.

**Preparation Example 2: Preparation of** metal layered **hydroxide complex using crossover interposition process**

As basic conditions for the experiment, all processes were performed at room temperature (20°C) and in a nitrogen atmosphere. Moreover, 95% ethanol and deionized water were used, and ascorbic acid (AA) and SMA, having a purity of 95% or more (water content of 5% or less), were used. A 3.2 M NaOH aqueous solution was prepared using deionized water.

SMA1 and SMA2, which are the surface modifiers used in the present invention, were pentapeptide-4 and palmitoyl dipeptide-7, respectively.

The experiment was carried out as follows in the order of synthesis, washing and drying. Specifically, 2.5 equivalent weights of ZnO were placed in a main tank, after which a concentrated HCl solution in an auxiliary tank 1 was slowly added dropwise to the main tank so that the pH was titrated to 0.5-1, followed by dissolution with stirring at 700 rpm for about 30 min in a nitrogen atmosphere. Thereafter, 1 equivalent weight of AA and 1 equivalent weight of SMA in an auxiliary tank 2, deionized water, and ethanol were placed in the main tank (the total volume ratio of the solvent that was added relative to Zn was 20) and were then dissolved with stirring at 300 rpm for about 10 min. Thereafter, the main tank was stirred, after which a 3.2 M NaOH solution in an auxiliary tank 3 was added to the main tank, so the pH in the main tank was titrated in the range of 6.5 to 7.5, after which a precipitation reaction was induced in the main tank with stirring for 3 hr. Here, the pH thereof was maintained at 6.5-7.5.

Next, the precipitate was washed. Specifically, in order to remove the unreacted salt in the above solution and the ionic material, impurities were removed using a centrifuge. The centrifugation process was performed three times for 5 min each at 8000 rpm, and the precipitate and the solution were separated from each other as follows. The synthesized precipitate solution was centrifuged at 8000 rpm for 5 min. After separation of the solution and the precipitate, the precipitate was uniformly diluted with ethanol and deionized water at a volume ratio of 1:1 and then centrifuged at 8000 rpm for 5 min. This procedure was repeated three times. In the final centrifugation washing process, the precipitate was diluted using ethanol alone and then centrifuged at 9000 rpm for 10 min, thus yielding a precipitate. The washed precipitate solution was spray dried with stirring at 600 rpm, thereby obtaining a powder.

Examples and Comparative Examples

Examples 1 to 20 and Comparative Examples 1 to 4

Metal layered hydroxide complexes were prepared in the same manner as in Preparation Example 1, with the exception that the amounts of the components were changed as shown in Tables 1 and 2 below.

[Table 1]

| Amount ratio (Molar ratio) | Example | | | | | | | | | | Comparative Example | |
|---|---|---|---|---|---|---|---|---|---|---|---|---|
| | 1 | 2 | 3 | 4 | 5 | 6 | 7 | 8 | 9 | 10 | 11 | 12 |
| SMA1 | 0.05 | 0.025 | 0.005 | 0.0025 | 0.0005 | 1 | 1 | 1 | 1 | 1 | 0 | 1 |
| AA | 1 | 1 | 1 | 1 | 1 | 0.05 | 0.025 | 0.006 | 0.0025 | 0.0005 | 1 | 0 |

[Table 2]

| Amount ratio (Molar ratio) | Example | | | | | | | | | | Comparative Example | |
|---|---|---|---|---|---|---|---|---|---|---|---|---|
| | 11 | 12 | 13 | 14 | 15 | 16 | 17 | 18 | 19 | 20 | 1 | 3 |
| SMA2 | 0.05 | 0.025 | 0.005 | 0.0025 | 0.0005 | 1 | 1 | 1 | 1 | 1 | 0 | 1 |
| AA | 1 | 1 | 1 | 1 | 1 | 0.05 | 0.025 | 0.005 | 0.0025 | 0.0005 | 1 | 0 |

Example 21 to 34 and Comparative Examples 1 to 3

Metal layered hydroxide complexes were prepared in the same manner as in Preparation Example 2, with the exception that the amounts of the components were changed as shown in Tables 3 and 4 below.

[Table 3]

| Amount ratio (Molar ratio) | Example | | | | | | | Comparative Example | |
|---|---|---|---|---|---|---|---|---|---|
| | 21 | 22 | 23 | 24 | 25 | 26 | 27 | 2 | 1 |
| SMA1 | 0.5 | 1 | 0.25 | 0.9995 | 0.995 | 0.005 | 0.0005 | 1 | 0 |
| AA | 0.5 | 0.25 | 0.75 | 0.0005 | 0.005 | 0.995 | 0.9995 | 0 | 1 |

[Table 4]

| Amount ratio (Molar ratio) | Example | | | | | | | Comparative Example | |
|---|---|---|---|---|---|---|---|---|---|
| | 28 | 29 | 30 | 31 | 32 | 33 | 34 | 3 | 1 |
| SMA2 | 0.5 | 1 | 0.25 | 0.9995 | 0.995 | 00.5 | 0.0005 | 1 | 0 |
| AA | 0.5 | 0.25 | 0.75 | 0.0005 | 0.005 | 0.995 | 0.9995 | 0 | 1 |

**Experimental Example 1. Measurement of XRD pattern**

**1-1. Measurement of XRD pattern and d-value of metal layered hydroxide prepared in Preparation Example 1**

**Analysis method 1: Powder X-ray diffractive pattern**

- Instrument: Powder X-ray Diffraction (PXRD)

X-ray diffractometer (D/MAXPRINT 2200-Ultima, Rigaku, Japan)

Cu-K$\alpha$ radiation ($\lambda$ = 1.5418 Å)

Tube voltage: 40 kV, current: 30 mA

As an X-ray diffractometer, a D/MAXPRINT 2200-Ultima, available from Rigaku (Japan), was used. The anode generating X-rays was formed of Cu metal, and measurement was performed using Ka rays ($\lambda$ = 1.5418 Å) at 2$\theta$ of 3 to 70°, a scanning speed of 0.02°/0.2 sec, and a divergence slit, scattering slit, and receiving slit of 0.1, 1, and 1 mm, respectively. The tube voltage was 40 kV, and the current was 30 mA.

- **Evaluation criterion**

The one-dimensional (1D) electron density for the z axis was calculated using Equation 1 below.

[Equation 1]

$$\rho(z) = \sum_{l=0}^{\infty} F_{00l} \cos \frac{2\pi l z}{c}$$

The powder obtained through synthesis was comparatively analyzed using an XRD pattern, and the interlayer distance was calculated using Bragg's equation (Equation 2 below). As for the foremost located peak, it represents the interlayer distance including the distance between the synthesized metal hydroxide layer and the anionic layer, which is regarded as a main interlayer distance.

[Equation 2]

$$n\lambda = 2d \sin \theta$$

($\lambda$ = X-ray wavelength, d = crystal lattice spacing, $\Theta$ = incident angle)

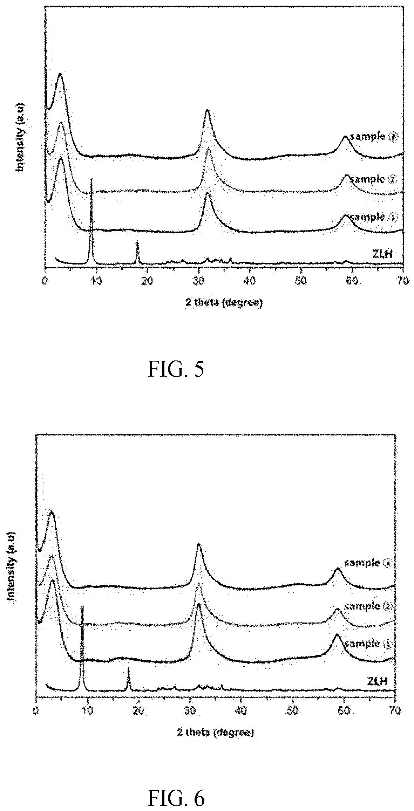

Experiments and evaluations were conducted using the same analysis method as in 1-1 above. In Table 5 and FIG. 5, Example 1 was represented as sample ①, Example 3 was represented as sample ②, and Example 5 was represented as sample ③, and in Table 6 and FIG. 6, Example 11 was represented as sample ①, Example 13 was represented as sample ②, and Example 15 was represented as sample ③. The specific values thereof are shown in Tables 5 and 6 (FIGS. 5 and 6).

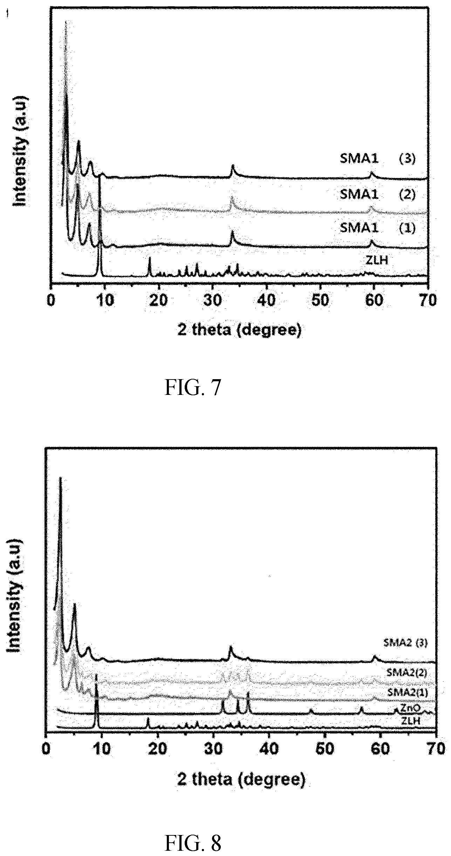

Also, in Table 7 and FIG. 7, Example 6 was represented as sample (1), Example 8 was represented as sample (2), and Example 10 was represented as sample (3), and in Table 8 and FIG. 8, Example 16 was represented as sample (1), Example 18 was represented as sample (2), and Example 20 was represented as sample (3). The specific values thereof are shown in Tables 7 and 8 (FIGS. 7 and 8).

**Reference Example: Preparation of zinc layered hydroxide**

5 g of $Zn(NO_3)_2 \cdot 6H_2O$ was dissolved in deionized water not containing carbonate ions ($CO_3^{2-}$) and then titrated to a pH of about 6 to 7 using 0.2 M NaOH, thus obtaining a zinc basic salt precipitate. The solution thus titrated was subjected to centrifugation and washing to remove unreacted salts, thereby obtaining 2.6 g of a white powder (yield: 70%).

[Table 5]

| sample | 2 theta (degree) | d-value (Å) |
|---|---|---|
| ZLH (Reference Example) | (002) 9.10<br>(004) 18.3 | 9.7 |
| sample ① (Example 1) | (002) 5.9 | 14.97 |
| sample ② (Example 3) | (002) 5.92 | 14.92 |
| sample ③ (Example 5) | (002) 5.94 | 14.86 |

[Table 6]

| sample | 2 theta (degree) | d-value (Å) |
|---|---|---|
| ZLH (Reference Example) | (002) 9.10<br>(004) 18.3 | 9.7 |
| sample 1 (Example 11) | (002) 6.04 | 14.62 |
| sample 2 (Example 13) | (002) 6.06 | 14.57 |
| sample 3 (Example 15) | (002) 5.96 | 14.81 |

As is apparent from the above tables, based on the results of surface modification by adding SMA1 or SMA2 based on the active ingredient (ascorbic acid) ratio, the $\Theta$ (theta) values of the main peak of each sample were 5.9, 5.92 and 5.94 when using SMA1 and were 6.04, 6.06 and 5.96 when using SMA2, and the d-values (interlayer distance values) were 14.97, 14.92 and 14.86 Å when using SMA1 and were 14.62, 14.57 and 14.81 Å when using SMA2, which were confirmed to be almost the same.

When using the outer ion-exchange process, it can be confirmed that SMA was not inserted into the interlayer spacing but was present on the surface.

[Table 7]

| sample | 2 theta (degree) | d-value (Å) |
|---|---|---|
| ZLH (Reference Example) | (002) 9.10<br>(004) 18.3 | 9.7 |
| sample (1) (Example 6)<br>sample (2) (Example 8) | (002) 2.25<br>(004) 4.43<br>(006) 6.61<br>(008) 8.83 | 39.8 |
| sample (3) (Example 10) | (002) 2.37<br>(004) 4.64<br>(006) 6.84<br>(008) 9.14 | 38.2 |

[Table 8]

| sample | 2 theta (degree) | d-value (Å) |
|---|---|---|
| ZLH (Reference Example) | (002) 9.10<br>(004) 18.3 | 9.7 |
| sample (1) (Example 16) | (002) 2.54<br>(004) 5.08<br>(006) 7.63 | 34.7 |
| sample (2) (Example 18) | (002) 2.59<br>(004) 5.18<br>(006) 7.775 | 34.08 |
| sample (3) (Example 20) | (002) 2.53<br>(004) 5.10<br>(006) 7.65 | 34.7 |

As is apparent from the above tables, based on the results of surface modification by adding ascorbic acid under the condition that the molar ratio of the inner component was 1 when the inner component was SMA, the $\Theta$ (theta) values of the main peak of each sample were 2.25, 2.25 and 2.37 when using SMA1 and were 2.54, 2.59 and 2.53 when using SMA2, and the d-values (interlayer distance values) were 39.8, 39.8 and 38.2 Å when using SMA1 and were 34.7, 34.08 and 34.7 Å when using SMA2, which were confirmed to be almost the same. This indicates that AA was not inserted into the interlayer spacing but was present on the surface.

**1-2. Measurement of XRD pattern and d-value of metal layered hydroxide prepared in Preparation Example 2**

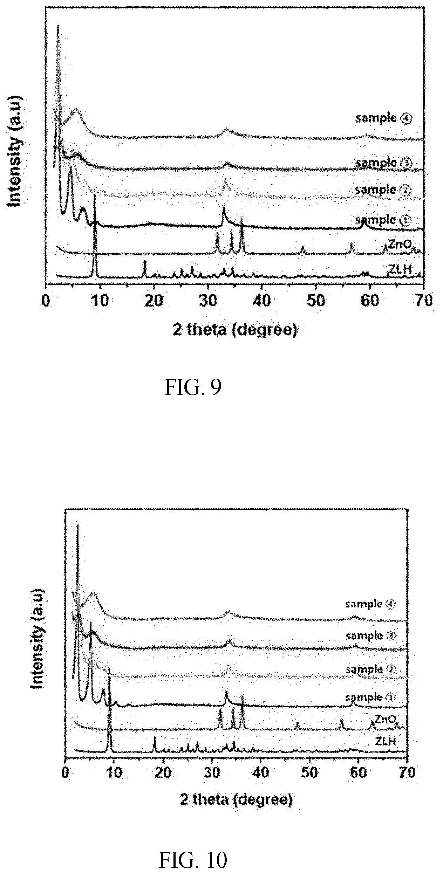

In Table 9 and FIG. 9, Comparative Example 5 was represented as sample ①, Example 21 was represented as sample ②, Example 22 was represented as sample ③ and Example 23 was represented as sample ④, and in Table 10 and FIG. 10, Comparative Example 7 was represented as sample ①, Example 28 was represented as sample ②, Example 29 was represented as sample ③ and Example 30 was represented as sample ④. The specific values thereof are shown in Tables 9 and 10 (FIGS. 9 and 10).

[Table 9]

| sample | 2 theta (degree) | d-value (Å) |
|---|---|---|
| ZLH | (002) 9.10<br>(004) 18.3 | 9.7 |

(continued)

| sample | 2 theta (degree) | d-value (Å) |
|---|---|---|
| sample ① | (002) 2.25<br>(004) 4.43<br>(006) 6.61<br>(008) 8.83 | 39.8 |
| sample ② | (002) 2.60<br>(004) 5.15<br>(006) 6.88 | 35.4 |
| sample ③ | (002) 2.84<br>(004) 5.82 | 30.7 |
| sample ④ | (002) 5.82 | 15.2 |

Description: XRD pattern after inner-outer surface treatment by controlling crystallinity after synthesis using SMA1 and VitC (ascorbic acid) at different ratios by crossover interposition process.

① SMA11 : VitC 0 ② SMA1 0.5 : VitC 0.5 ③ SMA1 0.25: VitC 0.75 ④ SMA1 0 : VitC 1

The change in XRD pattern after inner-outer surface treatment of ZLH-AA with SMA1 was evaluated. It was confirmed that the intensity of the XRD peak of the anion inserted into ZLH was changed depending on the change in the ratio of SMA1 and VitC based on the active ingredient.

When using SMA1 alone (1), values of 2.25, 4.43, 6.61 and 8.83 were observed, indicative of high crystallinity and well-aligned peak positions. Here, the interlayer spacing thereof was 39.8 Å. When the ratio of SMA1 and VitC was changed from sample 1 to 4, the interlayer distance varied, indicating that the position of the main peak, representing the SMA1 located in the layer, changed, and the effect of decreasing the interlayer distance in a gradual broad form appeared, and at the same time, a new large broad main peak of VitC was generated, and ultimately the SMA1 peak disappeared.

[Table 10]

| sample | 2 theta (degree) | d-value (Å) |
|---|---|---|
| ZLH | (002) 9.10<br>(004) 18.3 | 9.7 |
| sample ③ | (002) 2.59<br>(004) 5.21<br>(006) 7.73<br>(008) 10.42 | 34.1 |
| sample ② | (002) 2.76<br>(004) 5.39 | 32.4 |
| sample ③ | (002) 2.76<br>(004) 5.55 | 31.9 |
| sample ④ | (002) 5.82 | 15.2 |

Description: XRD pattern after inner-outer surface treatment by controlling crystallinity after synthesis using SMA2 and VitC (ascorbic acid) at different ratios by crossover interposition process.

① SMA2 1 : VitC 0 ② SMA2 0.5 : VitC 0.5 ③ SMA2 0.25: VitC 0.75 ④ SMA2 0 : VitC 1

The change in XRD pattern after inner-outer surface treatment of ZLH-AA with SMA2 was evaluated. It was confirmed that the intensity of the XRD peak of the anion inserted into ZLH was changed depending on the change in the ratio of SMA2 and VitC based on the active ingredient.

When using SMA2 alone (1), values of 2.59, 5.21, 7.73 and 10.42 were observed, indicative of high crystallinity and well-aligned peak positions. Here, the interlayer spacing thereof was 34.1 Å. When the ratio of SMA2 and VitC was changed from sample 1 to 4, the interlayer distance varied, indicating that the position of the main peak, representing the SMA2 located in the layer, changed, and the effect of decreasing the interlayer distance in a gradual broad form appeared, and at the same time, a new large broad main peak of VitC was generated, and ultimately the SMA2 peak disappeared.

**Experimental Example 2. Measurement of amounts of Zn, SMA1, SMA2 and AA**

**Analysis method 2: HPLC analysis**

- ASCORBIC ACID

Instrument: High-performance liquid chromatography (HPLC) analysis

Agilent 1100 series (Agilent Technologies, USA)

UV detector ($\lambda_{max}$ = 240)

Zorbax C18 column (4.6 mm x 150 mm, 5 $\mu$m, Agilent Technologies, USA)

Flow rate: 0.65 ml/min

Injection volume: 10 $\mu\ell$

Column temperature: 35°C

For HPLC analysis, an Agilent 1100 series (Agilent Technologies, USA) was used. $\lambda_{max}$ was measured at 240 nm, and measurement was performed using a Zorbax C18 column (4.6 mm x 150 mm, 5 $\mu$m, Agilent Technologies, USA) under conditions of 0.65 ml/min, an injection volume of 10 $\mu\ell$, and a column temperature of 35°C.
A mobile-phase buffer uses a solution of 0.1% trifluoroacetic acid (ReagentPlus®, 99%) in acetonitrile (anhydrous, 99.8%) and deionized water at a volume ratio of 2:8.
For sample treatment, 40 mg of a sample was mixed with 100 ml of a buffer solvent, sonicated for 10 min, and then rapidly stirred for 10 min. The resulting solution was filtered using a nylon syringe filter (pore size of 0.2 $\mu$m), and thus the sample was measured.

- SMA1 and SMA2

Instrument: High-performance liquid chromatography (HPLC) analysis

Agilent 1100 series (Agilent Technologies, USA)

UV detector ($\lambda_{max}$ = 230)

Luna 5u C18 100A (250 x 4.6 mm, 5 $\mu$m, Agilent Technologies, USA)

Flow rate: 1 ml/min

Injection volume: 10 $\mu\ell$

Column temperature: 30°C

For HPLC analysis, an Agilent 1100 series (Agilent Technologies, USA) was used. $\lambda_{max}$ was measured at 230 nm, and measurement was performed using a Luna 5u C 18 100A (250 x 4.6 mm, 5 $\mu$m, Agilent Technologies, USA) under conditions of 1 ml/min, an injection volume of 10 $\mu\ell$, and a column temperature of 30°C.
A mobile-phase buffer uses a solution of 0.1% trifluoroacetic acid (ReagentPlus®, 99%) in acetonitrile (anhydrous, 99.8%) and deionized water at a volume ratio of 5:5.
For sample treatment, 50 mg of a sample was mixed with 100 ml of a buffer solvent, sonicated for 10 min, and then rapidly stirred for 10 min. The resulting solution was filtered using a nylon syringe filter (pore size of 0.2 $\mu$m), and thus the sample was measured.

### 2-1: Measurement of amounts of Zn, SMA1, SMA2 and AA of metal layered hydroxide prepared in Preparation Example 1

The amounts of the components in Examples 1 to 10 and Comparative Examples 1 and 2 after the synthesis process were compared. This is an experiment to confirm the surface modification effect by the outer ion-exchange surface treatment process using VitC after synthesis of ZLH-SMA1 and ZLH-SMA2

[Table 11]

|  | SMA1 adding | AA adding | Zn(%) | SAM1 (%) | AA(%) |
|---|---|---|---|---|---|
| Example 1 | 0.05 | 1 | 35.958 | 6.34 | 38.75 |
| Example 2 | 0.025 | 1 | 37.140 | 3.23 | 40.02 |
| Example 3 | 0.005 | 1 | 38.144 | 0.64 | 41.1 |
| Example 4 | 0.0025 | 1 | 38.273 | 0.32 | 41.21 |
| Example 5 | 0.0005 | 1 | 38.377 | 0.068 | 41.34 |
| Comparative 1 Example | 0 | 1 | 38.403 | 0.000 | 41.37 |

[Table 12]

|  | SMA2 adding | AA adding | Zn(%) | SAM2 (%) | AA(%) |
|---|---|---|---|---|---|
| Example 11 | 0.05 | 1 | 36.448 | 5.05 | 39.15 |
| Example 12 | 0.025 | 1 | 37.400 | 2.51 | 40.01 |
| Example 13 | 0.005 | 1 | 38.198 | 0.51 | 41.06 |
| Example 14 | 0.0025 | 1 | 38.300 | 0.24 | 41.19 |
| Example 15 | 0.0005 | 1 | 38.382 | 0.05 | 41.28 |
| Comparative 1 Example | 0 | 1 | 38.403 | 0.000 | 41.37 |

When SMA1 or SMA2, the ratio of which was adjusted, was added, the amount of AA relative to the total weight was almost unchanged, and only the amount of SMA1 or SMA2 was changed. This confirms that AA exists unchanged between the metal salt layers and only SMA1 or SMA2 is attached to the surface. The slight change in the amount of AA corresponds to a numerical change caused by replacing the AA attached to the surface of the metal salt with SMA1 or SMA2. There was no peak change after SMA treatment on XRD, but modification with SMA was confirmed through measurement of amounts thereof.

[Table 13]

|  | SMA1 adding | AA adding | Zn(%) | SMA1(%) | AA(%) |
|---|---|---|---|---|---|
| Example 6 | 1 | 0.05 | 19.521 | 69.086 | 1.051 |
| Example 7 | 1 | 0.025 | 19.624 | 69.415 | 0.517 |
| Example 8 | 1 | 0.005 | 19.708 | 69.753 | 0.112 |
| Example 9 | 1 | 0.0025 | 19.717 | 69.82 | 0.049 |
| Example 10 | 1 | 0.0005 | 19.723 | 69.848 | 0.016 |
| Comparative 2 Example | 1 | 0 | 19.305 | 69.879 | 0.000 |

[Table 14]

|  | SMA2 adding | AA adding | Zn(%) | SMA2(%) | AA(%) |
|---|---|---|---|---|---|
| Example 16 | 1 | 0.05 | 22.679 | 63.751 | 1.234 |
| Example 17 | 1 | 0.025 | 22.939 | 64.194 | 0.617 |
| Example 18 | 1 | 0.005 | 23.005 | 64.397 | 0.130 |
| Example 19 | 1 | 0.0025 | 23.129 | 64.488 | 0.617 |
| Example 20 | 1 | 0.0005 | 23.137 | 64.569 | 0.039 |
| Comparative 3 Example | 1 | 0 | 23.150 | 64.637 | 0.000 |

When SMA was inserted between ZLH layers and then VitC, the ratio of which was adjusted, was added, the amount of SMA relative to the total weight was almost unchanged, and only the amount of AA was changed. This confirms that SMA exists unchanged between the metal salt layers and only AA is bound to the surface. The slight change in the amount of SMA corresponds to a numerical change caused by replacing the AA attached to the surface of the metal salt with SMA1 or SMA2 There was no peak change after SMA treatment on XRD, but modification with VitC was confirmed through measurement of amounts thereof.

**2-2: Measurement of amounts of Zn, SMA1, SMA2 and AA of metal layered hydroxide prepared in Preparation Example 2**

[Table 15]

|  | SMA1 adding | AA adding | Zn(%) | SMA1(%) | AA(%) |
|---|---|---|---|---|---|
| Comparative 2 Example | 1 | 0 | 19.305 | 69.879 | 0 |
| Example 24 | 0.9995 | 0.0005 | 19.635 | 69.71 | 0.01 |
| Example 25 | 0.995 | 0.005 | 19.654 | 69.53 | 0.098 |
| Example 21 | 0.5 | 0.5 | 26.036 | 46.11 | 14.021 |
| Example 23 | 0.25 | 0.75 | 29.989 | 27.34 | 25.035 |
| Example 26 | 0.005 | 0.995 | 33.121 | 0.658 | 40.73 |
| Example 27 | 0.0005 | 0.9995 | 38.219 | 0.065 | 41.19 |
| Comparative 1 Example | 0 | 1 | 38.403 | 0 | 41.37 |

[Table 16]

|  | SMA2 adding | AA adding | Zn(%) | SMA2(%) | AA(%) |
|---|---|---|---|---|---|
| Comparative 3 Example | 1 | 0 | 25.150 | 64.637 | 0 |
| Example 31 | 0.9995 | 0.0005 | 22.913 | 64.57 | 0.011 |
| Example 32 | 0.995 | 0.005 | 23.089 | 64.23 | 0.113 |
| Example 28 | 0.5 | 0.5 | 27.893 | 40.27 | 15.34 |
| Example 30 | 0.25 | 0.75 | 32.347 | 22.86 | 26.47 |
| Example 33 | 0.005 | 0.995 | 37.903 | 0.501 | 41.01 |
| Example 34 | 0.0005 | 0.9995 | 38.319 | 0.049 | 41.21 |
| Comparative 1 Example | 0 | 1 | 38.403 | 0 | 41.37 |

When SMA1 or SMA2, the ratio of which relative to the active ingredient was adjusted, was added, the amount of AA relative to the total weight was adjusted, and the amount of SMA1 or SMA2 was also adjusted. It was confirmed that the amounts were controlled by binding SMA1 or SMA2 between the metal salt layers together with AA

**Experimental Example 3. Measurement of Tyndall effect**

The Tyndall effect is based on the optical properties of a colloidal solution. The particles of the colloidal solution have a particle size of 1 nm to 1000 nm, and show that the particles are neither aggregated nor stabilized by Van der Waals attractions and electrostatic repulsion. Colloidal particles move rapidly in a dispersion medium, known as Brownian motion, and the size of colloidal particles is similar to or larger than the wavelength of light, so light cannot be reflected but light scattering occurs. Therefore, the Tyndall effect is a phenomenon that appears due to light scattering by colloidal particles.

In order to observe the Tyndall phenomenon, 50 mg of each powder was placed in a petri dish, which was then placed in a vial containing 50 ml of deionized water, followed by uniform dispersion for 10 min using a stirrer. All dispersion systems were set at 1000 ppm.

The solution was allowed to stand for 30 min, after which light was transmitted through a laser pointer beam to observe the Tyndall effect.

The case in which the particles in the solution were dispersed and the Tyndall effect was confirmed was determined to be transmission, and the case in which the particle size was 1 $\mu$m or more and thus precipitation occurred was determined to be precipitation.

**3-1 Measurement of Tyndall effect of metal layered hydroxide prepared in Preparation Example 1**

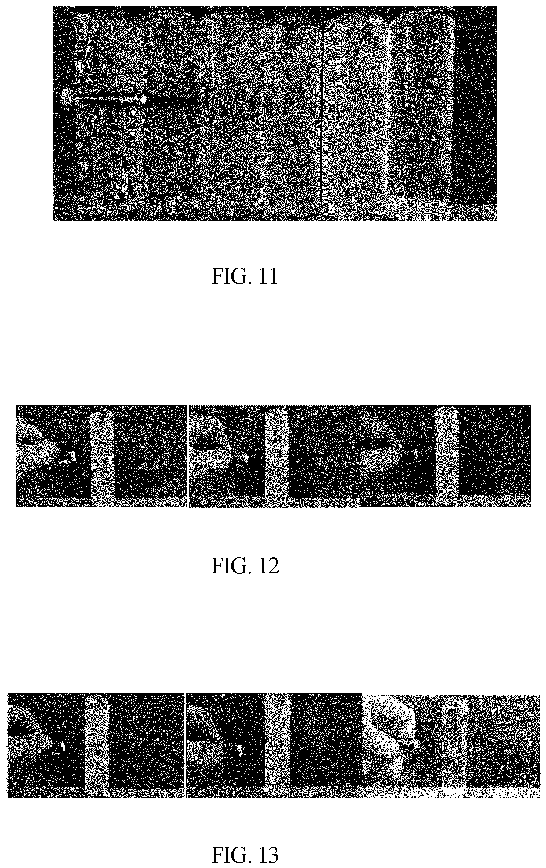

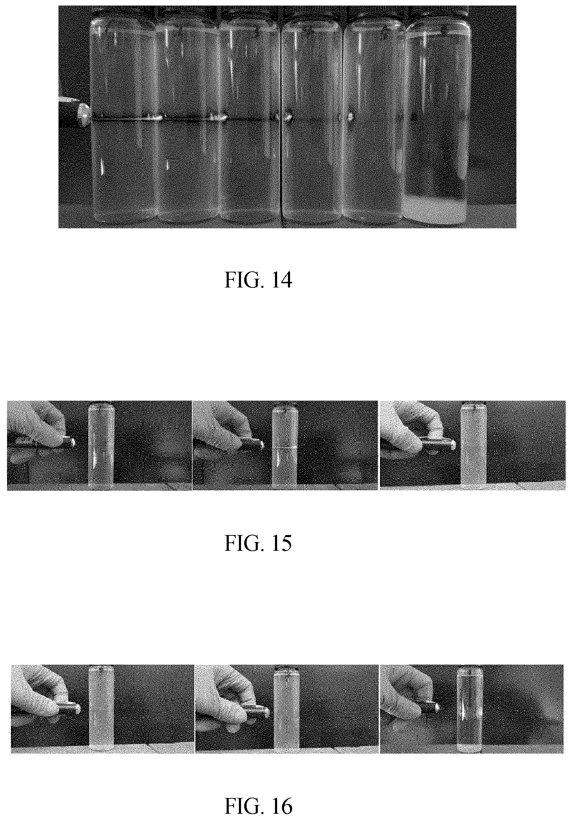

The Tyndall effect for each of the metal layered hydroxides prepared in Examples 1 to 5 and Comparative Example 1 is shown in FIGS. 11 to 13, and the Tyndall effect for each of the metal layered hydroxides prepared in Examples 11 to 15 and Comparative Example 2 is shown in FIGS. 14 to 16.

[Table 17]

| Classification | Sample | | | | | |
|---|---|---|---|---|---|---|
| | 1(Example 1) | 2(Example 2) | 3(Example3) | 4(Example 4) | 5(Example 5) | 6(Comarative EXample 1) |
| Tyndall effect | Transmission | Transmission | Transmission | Transmission | Transmission | Precipitation (transmission X) |

[Table 18]

| Classification | Sample | | | | | |
|---|---|---|---|---|---|---|
| | 1(Example 11) | 2(Example 12) | 3(Example 13) | 4(Example 14) | 5(Example 15) | 6(Comparative Example 1) |
| Tyndall effect | Transmission | Transmission | Transmission | Transmission | Transmission | Precipitation (transmission X) |

EP 3 760 183 A1

When the Tyndall effect occurred, it can be confirmed that dispersibility was excellent. In Comparative Example 1, which does not correspond to the present invention, it was confirmed that a precipitate was generated (FIGS. 11 to 16).

**3-2 Measurement of Tyndall effect of metal layered hydroxide prepared in Preparation Example** 2

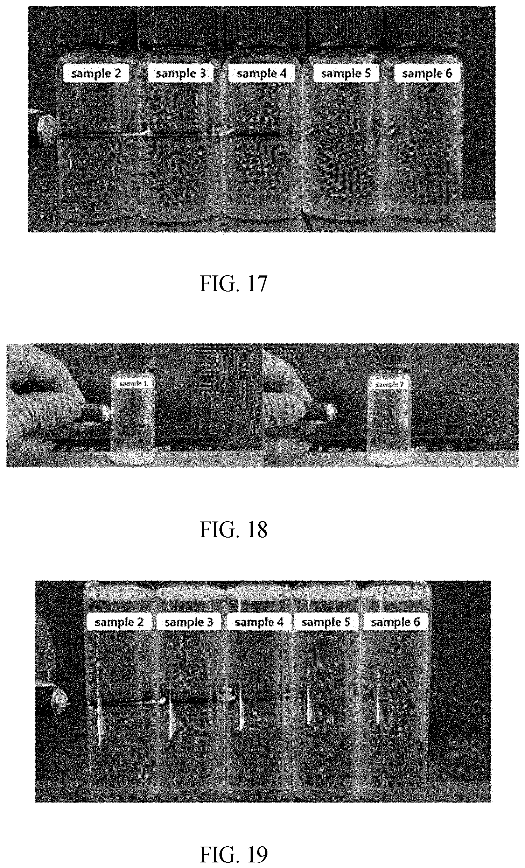

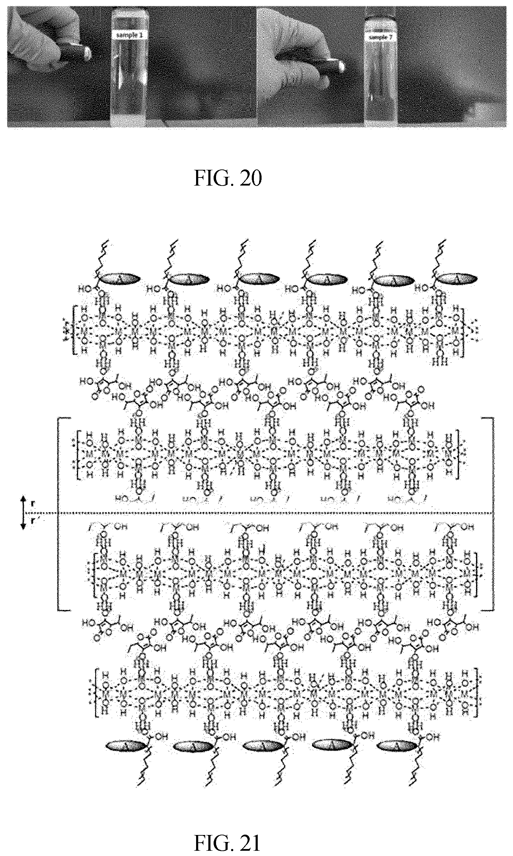

The Tyndall effect measured in the above experiment is shown in FIGS. 17 to 20.

When the Tyndall effect occurred, it can be confirmed that dispersibility was excellent. In Comparative Examples, which do not correspond to the present invention, it was confirmed that a precipitate was generated.

[Table 19]

| Classification | Sample | | | | | | |
|---|---|---|---|---|---|---|---|
| | 1<br>(Comparative Example 2) | 2<br>(Example 24) | 3<br>(Example 25) | 4<br>(Example 21) | 5<br>(Example 26) | 6<br>(Example 27) | 7<br>(Comparative Example 1) |
| Tyndall effect | Precipitation (transmission X) | transmission | transmission | transmission | transmission | transmission | Precipitation (transmission X) |

22

[Table 20]

| Classification | Sample | | | | | | |
|---|---|---|---|---|---|---|---|
| | 1 (Comparative Example 3) | 2 (Example 31) | 3 (Example 32) | 4 (Example 28) | 5 (Example 33) | 6 (Example 34) | 7 (Comparative Example 1) |
| Tyndall effect | Precipitation (transmission X) | transmission | transmission | transmission | transmission | transmission | Precipitation (transmission X) |

When the Tyndall effect occurred, it can be confirmed that dispersibility was excellent. In Comparative Examples, which do not correspond to the present invention, it was confirmed that a precipitate was generated (FIGS. 17 to 20).

**Experimental Example 4: Evaluation of particle size**

- Instrument: Zetasizer Nano ZS90

Dispersant agent: Water

DispersantRI: 1.330

Cell: Disposable cell

Run counts: 20

Temperature: 25°C

The particle size of the powder was measured using a Zetasizer available from Malvern Panalytical Ltd. (UK) using electrophoretic light scattering. The sample was dispersed in deionized water and then the particle size was measured using a NANO ZS ZS90.

**4-1: Evaluation of particle size of metal layered hydroxide prepared in Preparation Example 1**

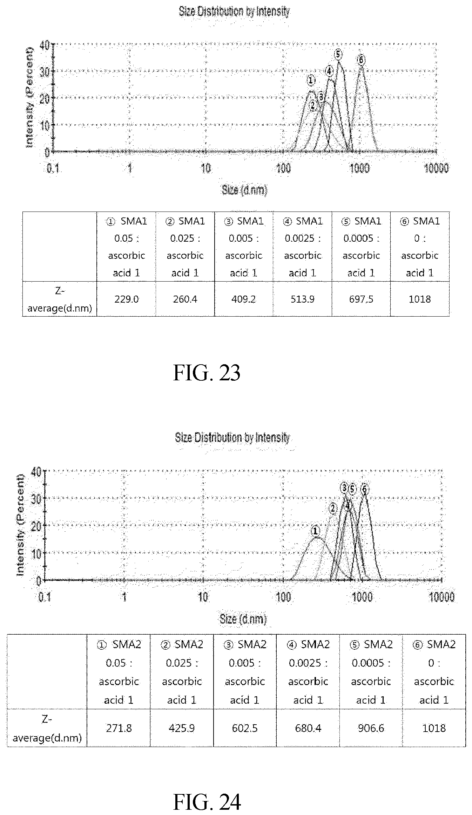

The particle sizes of Examples 1 to 5 and Comparative Example 1, measured by the above method, are specifically shown in Table 21 below, and the evaluation results thereof are shown in FIG. 23.

[Table 21]

| Classification | Sample | | | | | |
|---|---|---|---|---|---|---|
| | 1(Example 1) | 2(Example 2) | 3(Example 3) | 4(Example 4) | 5(Example 5) | 6 (Comparative Example 1) |
| Z-averave (d.nm) | 229.0 | 260.4 | 409.2 | 513.9 | 697.5 | 1018 |

The particle sizes of Examples 11 to 15 and Comparative Example 1, measured by the above method, are specifically shown in Table 22 below, and the evaluation results thereof are shown in FIG. 24.

[Table 22]

| Classification | Sample | | | | | |
|---|---|---|---|---|---|---|
| | 1(Example 11) | 2(Example 12) | 3(Example 13) | 4(Example 14) | 5(Example 15) | 6 (Comparative Example 1) |
| Z-averave(d.nm) | 271.8 | 425.9 | 602.5 | 680.4 | 906.6 | 1018 |

**4-2: Evaluation of particle size of metal layered hydroxide prepared in Preparation Example 2**

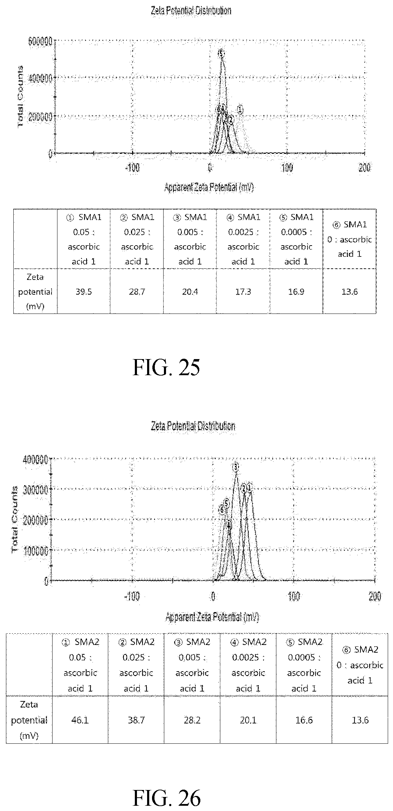

The particle sizes of Examples 21 and 24 to 27 and Comparative Example 1, measured by the above method, are specifically shown in Table 23 below, and the evaluation results thereof are shown in FIG. 25.

[Table 23]

| Classification | sample | | | | | |
|---|---|---|---|---|---|---|
| | 1 (Example 24) | 2 (Example 25) | 3 (Example 21) | 4 (Example 26) | 5 (Example 27) | 6 (Comparative Example 1) |
| Z-averave(d.nm) | 397.6 | 428.4 | 447.5 | 585.2 | 779.4 | 1018 |

The particle sizes of Examples 28 and 32 to 34 and Comparative Example 1, measured by the above method, are specifically shown in Table 24 below, and the evaluation results thereof are shown in FIG. 26.

[Table 24]

| Classification | Sample | | | | | |
|---|---|---|---|---|---|---|
| | 2 (Example 31) | 3 (Example 32) | 4 (Example 28) | 5 (Example 33) | 6 (Example 34) | 7 (Comparative Example 1) |
| Z-average(d.nm) | 231.7 | 444.1 | 513.9 | 625.8 | 798.7 | 1018 |

**Experimental Example 5: Analysis of zeta potential**

- Instrument: Zetasizer Nano ZS90

Dispersant agent: Water

Cell: Disposable zeta cell

Run counts: 30

Temperature: 25°C

The surface potential of the molecule was measured using a Zetasizer available from Malvern Panalytical Ltd. (UK) using electrophoretic light scattering. The sample was dispersed in deionized water and then the surface potential was measured using a NANO ZS ZS90.

**5-1: Analysis of zeta potential of metal layered hydroxide prepared in Preparation Example 1**

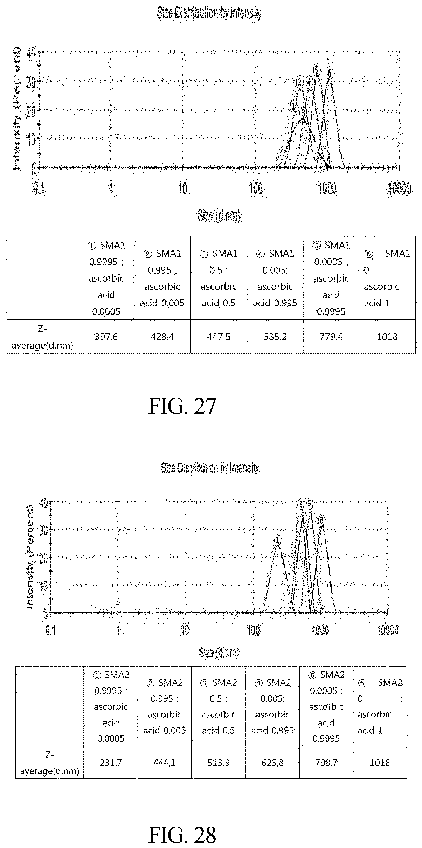

The zeta potential values of Examples 1 to 5 and Comparative Example 1, measured by the above method, are specifically shown in Table 25 below, and the specific graph thereof is shown in FIG. 27.

[Table 25]

| Classification | Sample | | | | | |
|---|---|---|---|---|---|---|
| | 1(Example 1) | 2(Example 2) | 3(Example 3) | 4(Example 4) | 5(Example 5) | 6 (Comparative Example 1) |
| Zeta potential (mV) | 39.5 | 28.7 | 20.4 | 17.3 | 16.9 | 13.6 |

The zeta potential values of Examples 11 to 15 and Comparative Example 1, measured by the above method, are specifically shown in Table 26 below, and the specific graph thereof is shown in FIG. 28.

[Table 26]

| Classification | Sample | | | | | |
|---|---|---|---|---|---|---|
| | 1(Example 11) | 2(Example 12) | 3(Example 13) | 4(Example 14) | 5(Example 15) | 6 (Comparative Example 1) |
| Zeta potential (mV) | 46.1 | 38.7 | 28.2 | 20.1 | 16.6 | 13.6 |

Based on the results measured upon adjusting the ratio of the active ingredient and the surface modifier, the zeta potential was increased compared to Comparative Example 1, based on which it was confirmed that the surface of the metal layered hydroxide was modified with the surface modifier. The zeta potential was increased compared to when using ascorbic acid of Comparative Example 1 or 3, which is a phenomenon whereby actual modification was caused by replacing ascorbic acid with SMA

**5-2: Analysis of zeta potential of metal layered hydroxide prepared in Preparation Example 2**

The zeta potential values of Examples 21 and 24 to 27 and Comparative Example 6, measured by the above method, are specifically shown in Table 27 below, and the evaluation results thereof are shown in FIG. 27.

[Table 27]

| Classfication | Sample | | | | | |
|---|---|---|---|---|---|---|
| | 1 (Example 24) | 2 (Example 25) | 3 (Example 21) | 4 (Example 26) | 5 (Example 27) | 6 (Comparative Example 1) |
| Z-average (d.n.m) | 397.6 | 428.4 | 447.5 | 585.2 | 779.4 | 1018 |

The zeta potential values of Examples 28 and 32 to 34 and Comparative Example 1, measured by the above method, are specifically shown in Table 28 below, and the evaluation results thereof are shown in FIG. 28.

[Table 28]

| Classification | Sample | | | | | |
|---|---|---|---|---|---|---|
| | 2 (Example 31) | 3 (Example 32) | 4 (Example 28) | 5 (Example 33) | 6 (Example 34) | 7 (Comparative Example 1) |
| Z-average:dnm) | 231.7 | 444.1 | 523.9 | 625.8 | 798.7 | 1018 |

**Experimental Example 6: Evaluation of controlled releasability**

For controlled releasability of each powder, the paddle method, which is most common among the general test methods of the Korean Pharmacopoeia, was used. 300 mg of each material powder was added to 300 ml of an aqueous solution of 0.08 wt% NaCl in deionized water (or a solution containing 0.08 wt% NaCl and 10% ethanol) and dispersed therein, after which the release of a measurement component was induced with stirring at 25°C at 50 rpm, and the solution was sampled at a predetermined time interval, filtered using a nylon syringe filter (pore size of 0.2 $\mu$m), and measured using HPLC.

6-1: **Evaluation of controlled releasability of metal layered hydroxide prepared in Preparation Example 1**

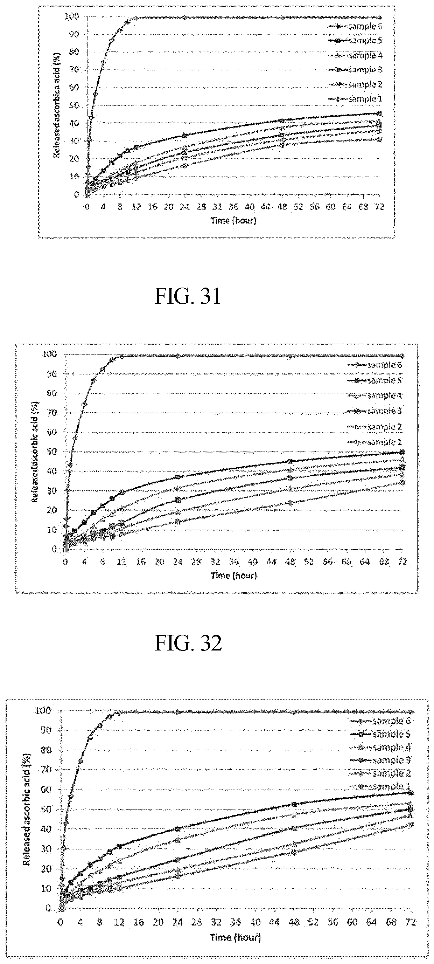

The specific values related to controlled releasability of Examples 1 to 5 and Comparative Example 1, measured by the above method, are shown in Table 29 below, and the specific graph thereof is shown in FIG. 31.

[Table 29]

| Classification | Sample | | | | | |
|---|---|---|---|---|---|---|
| | 1(Example 1) | 2(Example 2) | 3(Example 3) | 4(Example 4) | 5(Example 5) | 6(Comparative Example 1) |
| Cumulative AA release (%) | 31.18 | 35.69 | 38.94 | 41.12 | 45.54 | 99.19 |

Depending on the ratio of the surface modifier in Examples, the controlled releasability of ascorbic acid was increased. It was confirmed that the controlled releasability increased from a minimum of about 53% to a maximum of about 68% for 72 hr depending on the ratio of the surface modifier, and also that the controlled releasability of the crystal, in which the amount of the surface modifier was lower than the amount of ascorbic acid, was significantly improved with an increase in the storage period.

The specific values related to controlled releasability of Examples 11 to 15 and Comparative Example 1, measured by the above method, are shown in Table 30 below, and the specific graph thereof is shown in FIG. 32.

[Table 30]

| Classification | Sample | | | | | |
|---|---|---|---|---|---|---|
| | 1(Example 11) | 2(Example 12) | 3(Example 13) | 4(Example 14) | 5(Example 15) | 6Comarative Example 1) |
| Cumulative AA release (%) | 422 | 5.52 | 7.85 | 9.12 | 5.91 | 51.53 |

Depending on the ratio of the surface modifier in Examples, the controlled releasability of ascorbic acid was increased. It was confirmed that the controlled releasability increased from a minimum of about 49% to a maximum of about 64% for 72 hr depending on the ratio of the surface modifier, and also that the controlled releasability of the crystal, in which the amount of the surface modifier was lower than the amount of ascorbic acid, was significantly improved with an increase in the storage period.

**6-2: Evaluation of controlled releasability of metal layered hydroxide prepared in Preparation Example 2**

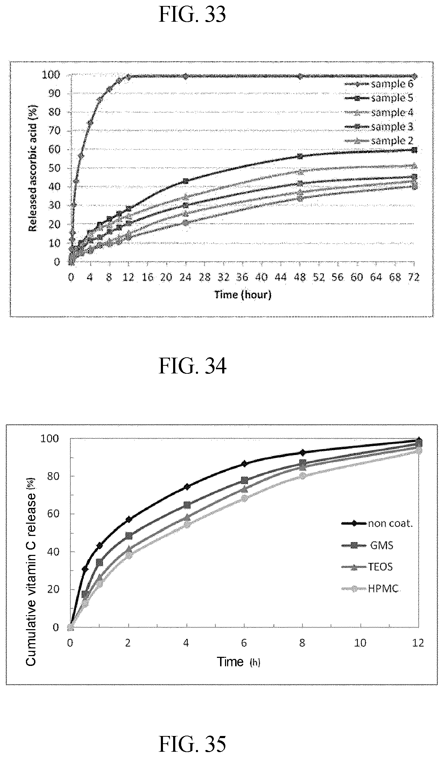

The specific values related to controlled releasability of Examples 21 and 24 to 27 and Comparative Example 1, measured by the above method, are shown in Table 31 below, and the evaluation results thereof are shown in FIG. 33.

[Table 31]

| Classification | Sample | | | | | |
|---|---|---|---|---|---|---|
| | 1 (Example 24) | 2 (Example 25) | 3 (Example 21) | 4 (Example 26) | 5 (Example 27) | 6(Comarative Example 1) |
| Cumulative AA release (%) | 40.24 | 43.24 | 45.98 | 51.59 | 59.84 | 91.19 |

Depending on the ratio of the surface modifier in Examples, the controlled releasability of ascorbic acid was increased. It was confirmed that the controlled releasability increased from a minimum of about 40% to a maximum of about 57% for 72 hr depending on the ratio of the surface modifier, and also that the controlled releasability of the crystal, in which the amount of the surface modifier was lower than the amount of ascorbic acid, was significantly improved with an increase in the storage period.

The specific values related to controlled releasability of Examples 28 and 31 to 34 and Comparative Example 1, measured by the above method, are shown in Table 32 below, and the evaluation results thereof are shown in FIG. 34.

[Table 32]

| Classification | Sample | | | | | |
|---|---|---|---|---|---|---|
| | 2 (Example 31) | 3 (Example 32) | 4 (Example 28) | 5 (Example 33) | 6 (Example 34) | 7(Comparative Example 1) |
| Cumulative AA release (%) | 11.69 | 19.93 | 25.94 | 31.28 | 37.28 | 51.53 |

Depending on the ratio of the surface modifier in Examples, the controlled releasability of ascorbic acid was increased. It was confirmed that the controlled releasability increased from a minimum of about 39% to a maximum of about 58% for 72 hr depending on the ratio of the surface modifier, and also that the controlled releasability of the crystal, in which the amount of the surface modifier was lower than the amount of ascorbic acid, was significantly improved with an increase in the storage period.

**Experimental Example 7: Evaluation of particle size and zeta potential when using conventional coating agent**

[Table 33]

| | | | particle size(nm) | zeta potential(m v) |
|---|---|---|---|---|
| Item | Repeat. | Repeat. | water | water |
| Comparative Example 4 | 1 | 1 | 9058.10 | -16.43 |
| | 2 | 2 | 10987.40 | -25.22 |
| HPMC(hydroxypropyl methylcellulose) coating | Aver. | Aver. | 10022.75 | -20.83 |
| | | | particle size(nm) | zeta potential(m v) |
| Item | Repeat. | Repeat. | water | water |
| Comparative Example 5 | 1 | 1 | 7073.10 | -7.01 |
| | 2 | 2 | 7520.30 | -7.20 |
| TEOS coating | Aver. | Aver. | 7296.70 | -7.11 |
| | | | particle size(nm) | zeta potential(m v) |
| Item | Repeat. | Repeat. | water | water |
| Comparative Example 6 | 1 | 1 | 3697.70 | -4.84 |
| | 2 | 2 | 3597.80 | -4.84 |
| GMS coating | Aver. | Aver. | 3647.75 | -4.84 |

After coating with conventional HPMC, TEOS and GMS, the particle size was generally measured to be 3 $\mu$m (3000 nm) or more, which is evaluated to be greater than the particle size of the present invention. The zeta potential has a negative value, which is the result obtained for the coating agent that was used. The relatively small particle size is advantageous for skin absorption.

**Experimental Example 8: Evaluation of controlled releasability**

The controlled releasability of Comparative Examples 4 to 6 and non-coated ZBS-Vitamin C (Comparative Example 1) was evaluated. The specific results thereof are shown in FIG. 35.

As shown in FIG. 35, the controlled releasability of the metal layered hydroxide complex coated with a conventional coating agent reached 100% after 12 hr. Specifically, it was confirmed that the controlled releasability when using the conventional coating agent was very low compared to the present invention.

**Experimental Example 9: Confirmation of yield and amount of Examples 1 to 34 and Comparative Examples 1 to 3**

[Table 34]

| | material | | equiv. | Example 1 usage (g) | Example 2 usage (g) | Example 3 usage (g) | Example 4 usage (g) | Example 5 usage (9) | Comparative Example 1 usage (g) |
|---|---|---|---|---|---|---|---|---|---|
| 1st | ZnO | | 2.5 | 5 | 5 | 5 | 5 | 5 | 5 |
| | conc. HCl(pH) | | | 0.8 | 0.78 | 0.79 | 0.82 | 0.81 | 0.81 |
| | solvent | EtOH(mL) | | 40 | 40 | 40 | 40 | 40 | 40 |
| | | H2O(mL) | | 40 | 40 | 40 | 40 | 40 | 40 |
| | | subtotal(mL) | | 100 | 100 | 100 | 100 | 100 | 100 |
| 2nd | ascorbic acid | | 1 | 4.33 | 4.33 | 4.33 | 4.33 | 4.33 | 4.33 |
| | solvent | EtOH(mL) | | 10 | 10 | 10 | 10 | 10 | 10 |
| | | H2O(mL) | | 10 | 10 | 10 | 10 | 10 | 10 |
| | | subtotal(mL) | | 20 | 20 | 20 | 20 | 20 | 20 |
| 3rd | 32M NaOH | | | 30.7 | 30.6 | 30.6 | 30.7 | 30.6 | 30.6 |
| 4th | washing step | | | | | | | | |
| 5th | SMA1(g) | | | 0.711 | 0.355 | 0.071 | 0.035 | 0.07 | . |
| | solvent | EtOH(mL) | | 20 | 20 | 20 | 20 | 20 | |
| | | H2O(mL) | | 20 | 20 | 20 | 20 | 20 | |
| 6th | washing step | | | | | | | | |
| Final | | | | | | | | | |
| result | yeld(g) | | | 8.24 | 8.16 | 8.12 | 8.12 | 8.13 | 8.13 |
| | yield(%) | | | 73.77 | 75.41 | 77.08 | 77.35 | 77.62 | 77.74 |
| | color | | | white off | white off | white off | off | white off | white off |
| | content(%) | Zn(%) | | 35.958 | 37.140 | 38.144 | 38.273 | 38.377 | 38.403 |
| | | SMA1 | | 6.34 | 3.23 | 0.64 | 0.32 | 0.06 | 0.00 |
| | | ascorbic acid | | 38.75 | 40.02 | 41.10 | 41.21 | 41.34 | 41.57 |

[Table 35]

| | | material | equiv. | Example 6 usage (g) | Example 7 usage (g) | Example 8 usage (g) | Example 9 usage (g) | Example 10 usage (g) | Comparative Example 2 usage (g) |
|---|---|---|---|---|---|---|---|---|---|
| 1st | | ZnO | 2.5 | 5 | 5 | 5 | 5 | 5 | 5 |
| | | conc. HCl(pH) | | | | | | | |
| | solvent | EtOH(mL) | | 40 | 40 | 40 | 40 | 40 | 40 |
| | | H2O(mL) | | 40 | 40 | 40 | 40 | 40 | 40 |
| | | subtotal(mL) | | 100 | 100 | 100 | 100 | 100 | 100 |
| 2nd | | **SMA1** | **1** | **14.224** | **14.224** | **14.224** | **14.224** | **14.224** | **14.224** |
| | solvent | EtOH(mL) | | 10 | 10 | 10 | 10 | 10 | 10 |
| | | H2O(mL) | | 10 | 10 | 10 | 10 | 10 | 10 |
| | | subtotal(mL) | | 20 | 20 | 20 | 20 | 20 | 20 |
| 3rd | | 3.2M NaOH | | 30.6 | 30.7 | 30.6 | 30.6 | 30.5 | 30.6 |
| 4th | | washing step | | | | | | | |
| 5th | | **ascorbic acid** | | **0.216** | **0.108** | **0.022** | **0.011** | **0.002** | . |
| | solvent | EtOH(mL) | | 20 | 20 | 20 | 20 | 20 | |
| | | H2O(mL) | | 20 | 20 | 20 | 20 | 20 | |
| 6th | | washing step | | | | | | | |
| Final | | | | | | | | | |
| result | | yield(g) | | 6.58 | 6.61 | 6.69 | 6.70 | 6.76 | 6.89 |
| | | yield(%) | | 31.99 | 32.31 | 32.83 | 32.93 | 33.21 | 33.86 |
| | | color | | white off | white off | white off | white off | white off | white off |
| | content(%) | Zn(%) | | 19.521 | 19.624 | 19.708 | 19.717 | 19.723 | 19.305 |
| | | SMA1 | | 69.086 | 69.415 | 69.753 | 69.82 | 69.848 | 69.879 |
| | | ascorbic acid | | 1.051 | 0.517 | 0.112 | 0.049 | 0.016 | 0 |

[Table 36]

| | material | | equiv. | Example 11 | Example 12 | Example 13 | Example 14 | Example 15 | Comparative Example 1 |
|---|---|---|---|---|---|---|---|---|---|
| | | | | usage (g) | usage (g) | usage (g) | usage (g) | usage (g) | usage (g) |
| 1st | ZnO | | 2.5 | 5 | 5 | 5 | 5 | 5 | 5 |
| | conc. HCl(pH) | | | 0.8 | 0.8 | 0.81 | 0.79 | 0.81 | 0.8 |
| | solvent | EtOH(mL) | | 40 | 40 | 40 | 40 | 40 | 40 |
| | | H2O(mL) | | 40 | 40 | 40 | 40 | 40 | 40 |
| | | subtotal(mL) | | 100 | 100 | 100 | 100 | 100 | 100 |
| 2nd | ascorbic acid | | 1 | 4.33 | 4.33 | 4.33 | 4.33 | 4.33 | 4.33 |
| | solvent | EtOH(mL) | | 10 | 10 | 10 | 10 | 10 | 10 |
| | | H2O(mL) | | 10 | 10 | 10 | 10 | 10 | 10 |
| | | subtotal(mL) | | 20 | 20 | 20 | 20 | 20 | 20 |
| 3rd | 3.2M NaOH | | | 30.6 | 30.6 | 30.6 | 30.6 | 30.7 | 30.6 |
| 4th | washing step | | | | | | | | |
| 5th | SMA2 (g) | | | 0.561 | 0.281 | 0.056 | 0.028 | 0.05 | . |
| | solvent | EtOH(mL) | | 20 | 20 | 20 | 20 | 20 | |
| | | H2O(mL) | | 20 | 20 | 20 | 20 | 20 | |
| 6th | washing step | | | | | | | | |
| Final | | | | | | | | | |
| result | yield(g) | | | 7.94 | 7.96 | 8.06 | 8.17 | 8.11 | 8.13 |
| | yield(%) | | | 72.04 | 74.07 | 76.63 | 77.88 | 77.48 | 77.74 |
| | color | | | white off | white off | white off | white off | white off | white off |
| | content(%) | Zn(%) | | 36.448 | 37.400 | 38.198 | 38.300 | 33.382 | 33.403 |
| | | SMA2 | | 5.05 | 2.51 | 0.51 | 0.24 | 0.05 | 0.00 |
| | | ascorbic acid | | 39.15 | 40.01 | 41.06 | 41.19 | 41.28 | 41.37 |

[Table 37]

| | | material | equiv. | Example 16 | Example 17 | Example 18 | Example 19 | Example 20 | Comparative Example 3 |
|---|---|---|---|---|---|---|---|---|---|
| | | | | usage (g) | usage (g) | usage (g) | usage (g) | usage (g) | usage (g) |
| 1st | | ZnO | 2.5 | 5 | 5 | 5 | 5 | 5 | 5 |
| | | conc. HCl(pH) | | | | | | | |
| | solvent | EtOH(mL) | | 40 | 40 | 40 | 40 | 40 | 40 |
| | | H2O(mL) | | 40 | 40 | 40 | 40 | 40 | 40 |
| | | subtotal(mL) | | 100 | 100 | 100 | 100 | 100 | 100 |
| 2nd | | **SMA2** | **1** | **11.219** | **11.219** | **11.219** | **11.219** | **11.219** | **11.219** |
| | solvent | EtOH(mL) | | 10 | 10 | 10 | 10 | 10 | 10 |
| | | H2O(mL) | | 10 | 10 | 10 | 10 | 10 | 10 |
| | | subtotal(mL) | | 20 | 20 | 20 | 20 | 20 | 20 |
| 3rd | | 3.2M NaOH | | 30.6 | 30.6 | 30.7 | 30.6 | 30.6 | 30.6 |
| 4th | | washing step | | | | | | | |
| 5th | | ascorbic acid | | 0.561 | 0.281 | 0.056 | 0.028 | 0.005 | |
| | solvent | EtOH(mL) | | 20 | 20 | 20 | 20 | 20 | |
| | | H2O(mL) | | 20 | 20 | 20 | 20 | 20 | |
| 6th | | washing step | | | | | | | |
| Final | | | | | | | | | |
| result | | yield(g) | | 3.29 | 3.12 | 2.98 | 2.91 | 2.30 | 2.74 |
| | | yield(%) | | 18.73 | 17.89 | 17.15 | 16.78 | 16.11 | 15.81 |
| | | color | | white off | white off | white off | white off | white off | white off |
| | content(%) | Zn (%) | | 22.679 | 22.939 | 23.005 | 23.129 | 23.137 | 23.150 |
| | | SMA2 | | 63.751 | 64.194 | 64.397 | 64.488 | 64.569 | 64.637 |
| | | ascorbic acid | | 1.234 | 0.617 | 0.13 | 0.617 | 0.039 | 0 |

EP 3 760 183 A1

[Table 38]

| | material | | equiv. | Comparative Example 2 | Example 24 | Example 25 | Example 21 | Example 23 | Example I 26 | Example 27 | Comparative Example 1 |
|---|---|---|---|---|---|---|---|---|---|---|---|
| | | | | usage (g) | usage (g) | usage (g) | usage (g) | usage (g) | usage (g) | usage (g) | usage (9) |
| 1st | ZnO | | 2.5 | 5 | 5 | 5 | 5 | 5 | 5 | 5 | 5 |
| | conc. HCl(pH) | | | 0.8 | 0.8 | 0.8 | 0.81 | 0.8 | 0.81 | | 0.8 |
| | solvent | EtOH | | 40 | 40 | 40 | 40 | 40 | 40 | 40 | 40 |
| | | H2O | | 40 | 40 | 40 | 40 | 40 | 40 | 40 | 40 |
| | | subtotal | | 80 | 80 | 80 | 80 | 80 | 80 | 100 | 100 |
| 2nd | SMA1 | | 1 | 14.224 | 14.21 7 | 14.15 3 | 7.112 | 3.556 | 0.071 | 0.007 | - |
| | ascorbic acid | | | - | 0.002 | 0.022 | 2.164 | 3.246 | 4.307 | 4.326 | 4.330 |
| | solvent | EtOH | | 10 | 10 | 10 | 10 | 10 | 10 | 10 | 10 |
| | | H2O | | 10 | 10 | 10 | 10 | 10 | 10 | 10 | 10 |
| | | subtotal | | 20 | 20 | 20 | 20 | 20 | 20 | 20 | 20 |
| 3rd | 3.2M NaOH | | | 30.6 | 30.6 | 30.6 | 30.7 | 30.6 | 30.7 | 30.7 | 30.7 |
| 4th | washing step | | | | | | | | | | |
| resul t | yield(g) | | | 6.89 | 6.95 | 7.25 | 8.08 | 8.26 | 8.02 | 8.03 | 8.13 |
| | yield(%) | | | 33.86 | 34.16 | 35.71 | 52.45 | 63.89 | 76.27 | 77.74 | 77.74 |
| | color | | | white off | white off | white off | white off | white off | white off | white off | white off |
| | content (%) | Zn (%) | | 19.305 | 19.63 5 | 19.65 4 | 26.03 6 | 29.98 9 | 38.12 1 | 38.219 | 38.403 |
| | | SMA1 | | 69.379 | 69.71 | 69.53 | 46.11 | 27.34 | 0.658 | 0.065 | 0.00 |
| | | ascorbi acid | | 0 | 0.01 | 0.098 | 14.02 1 | 25.03 5 | 40.73 | 41.19 | 4137 |

[Table 39]

| | material | | equiv. | Comparative Example 3 | Example 31 | Example 32 | Example 28 | Example 30 | Example 33 | Example 34 | Comparative Example 1 |
|---|---|---|---|---|---|---|---|---|---|---|---|
| | | | | usage (g) | usage (g) | usage (g) | usage (g) | usage (g) | usage (g) | usage (g) | usage (g) |
| 1st | ZnO | | 2.5 | 5 | 5 | 5 | 5 | 5 | 5 | 5 | 5 |
| | conc. HCl(pH) | | | | 0.81 | 0.81 | 0.8 | 0.8 | 0.81 | | 0.8 |
| | solvent | EtOH | | 40 | 40 | 40 | 40 | 40 | 40 | 40 | 40 |
| | | H2O | | 40 | 40 | 40 | 40 | 40 | 40 | 40 | 40 |
| | | subtotal | | 80 | 80 | 80 | 80 | 80 | 80 | 100 | 100 |
| 2nd | SMA2 | | 1 | 11.219 | 11.21 3 | 11.163 | 5.610 | 2.805 | 0.056 | 0.060 | - |
| | ascorbic acid | | | 0.000 | 0.002 | 0.022 | 2.164 | 3.246 | 4.307 | 4.326 | 4.330 |
| | solvent | EtOH | | 10 | 10 | 10 | 10 | 10 | 10 | 10 | 10 |
| | | H2O | | 10 | 10 | 10 | 10 | 10 | 10 | 10 | 10 |
| | | subtotal | | 20 | 20 | 20 | 20 | 20 | 20 | 20 | 20 |
| 3rd | 3.2M NaOH | | | 30.7 | 30.7 | 30.7 | 30.6 | 30.6 | 30.7 | 30.7 | 30.7 |
| 4th | washing step | | | | | | | | | | |
| result | yield(g) | | | 2.74 | 2.88 | 3.13 | 5.94 | 7.77 | 7.83 | 7.99 | 8.13 |
| | yield(g) | | | 15.81 | 16.58 | 18.06 | 42.68 | 63.79 | 74.60 | 77.74 | 77.74 |
| | color | | | white off | white off | white off | white off | white off | white off | white off | white off |
| | content (% ) | Zn (%) | | 23.150 | 22.91 3 | 23.039 | 27.893 | 32.34 7 | 37.90 3 | 38.31 9 | 38.403 |
| | | SMA1 | | 64.637 | 64.57 | 64.28 | 40.27 | 22.86 | 0.501 | 0.049 | 0.00 |
| | | ascorbi c acid | | 0 | 0.011 | 0.113 | 15.34 | 26.47 | 41.01 | 41.21 | 41.37 |

**Industrial Applicability**

According to the present invention, a metal layered hydroxide complex includes an active ingredient and a surface modifier, thus exhibiting superior controlled releasability. The metal layered hydroxide complex has a reduced particle size, in which the active ingredient is contained stably therein. Moreover, the metal layered hydroxide complex can be simply synthesized in a manner in which a metal layered hydroxide is bound to an active ingredient having a charge opposite thereto through electrostatic attraction or in which the crystallinity of a metal layered hydroxide is adjusted, thus achieving a short processing time and obviating a dedicated preparation facility, thereby generating environmentally friendly and economical effects.

In addition, the present invention provides a method of preparing metal layered hydroxide, which obviates post-treatment such as washing due to the use of an organic solvent, and thus is safe without the use of harmful materials.

**Claims**

1.   A metal layered hydroxide complex comprising an active ingredient and a surface modifier,
     wherein the active ingredient is at least one selected from the group consisting of ascorbic acid, biotin, pantothenic acid, cysteine, ferulic acid, glutamic acid, indole acetic acid, cinnamic acid, caffeic acid, thiamine, riboflavin, niacin, pantothenic acid, pyridoxine, folic acid, calciferol
     tocopherol, tranexamic acid, salicylic acid, retinoic acid and arbutin, and
     the surface modifier comprises at least one selected from the group consisting of a peptide containing 2 to 8 amino acids and a peptide/fatty-acid conjugate comprising a peptide containing 2 to 8 amino acids and a fatty acid containing 10 to 16 carbon atoms.

2.   The metal layered hydroxide complex of claim 1, wherein the peptide is at least one selected from the group consisting of dipeptide-1 (YR), dipeptide-2 (VW), dipeptide-4 (FW), dipeptide-6 (KV), dipeptide-7(KT), dipeptide-14 (AT), dipeptide (GH), acetyl dipeptide-1 (YR), acetyl dipeptide-1 cetyl Ester (YR), nicotinoyl dipeptide-2 (VW), CP dipeptide (CP), VGE dipeptide (VE), CGE dipeptide (CE), EGE dipeptide (EE), TGE dipeptide (TE), LGE dipeptide (LE), EQ dipeptide (EQ), GR dipeptide (GR), HG dipeptide (HG), PE dipeptide (PE), DE dipeptide (DE), HQ dipeptide (HQ), RS dipeptide (RS), HP dipeptide (HP), carnosine (AH), tripeptide-1 (GHK), tripepdide-3 (GHR), tripeptide-4 (LGD), tripeptide-5 (KVK), tripeptide-6 (GXP), tripeptide-8 (HFR), tripeptide-10 (KDI), RGD peptide (RGD), AHK peptide (AHK), tripeptide-29 (GPX), tripeptide-54 (FTY), biotinoyl tripeptide-1 (GHK), thioctoyl tripeptide-1 (GHK), tripeptide (RFK), HGG peptide (HGG), peptide CK (RKR), tetrapeptide-1 (LPTV), tetrapeptide-2 (KDVY), tetrapeptide-3 (KGHK), tetrapeptide-5 (AHSH), tetrapeptide-7 (GQPR), tetrapeptide-9 (QDVH), tetrapeptide-11 (PPYL), tetrapeptide-15 (YPFF), tetrapeptide-21 (GEKG), tetrapeptide-26 (ELPS), acetyl tetrapeptide-2 (KDVY), acetyl tetrapeptide-3 (KGHK), acetyl tetrapeptide-5 (AHSH), acetyl tetrapeptide-9 (QDVH), acetyl tetrapeptide-11 (PPYL), acetyl tetrapeptide-15 (YPFF), pentapeptide-3 (GPRPA), pentapeptide-4 (KTTKS), pentapeptide-17 (KLAKK), pentapeptide-18 (YAGFL), thioctoyl pentapeptide-4 (KTTKS), hexapeptide-1 (ARHLFW), hexapeptide-2 (FWFKPV), hexapeptide-3 (EEMQRR), hexapeptide-4 (FGHXAF), hexapeptide-5 (FGVXAF), hexapeptide-6 (VEPIPY), hexapeptide-9 (GPQGPQ), hexapeptide-11 (FVAPFP), hexapeptide-12 (VGVAPG), acetyl hexapeptide-1 (ARHLFW), acetyl hexapeptide-3 (EEMQRR), acetyl hexapeptide-6 (VEPIPY), heptapeptide-1 (EDDDWDF), heptapeptide-7 (MGRNIRN), cysteine peptide (RFAACAA), lysine peptide (RFAAKAA), selank (TKPRPGP), octapeptide-2 (TAEEHEVM), octapeptide-3 (EEMQRRAD), octapeptide-4 (YGGFLGHK) and acetyl octapeptide-3 (EEMQRRAD).

3.   The metal layered hydroxide complex of claim 1, wherein the peptide/fatty-acid conjugate is at least one selected from the group consisting of palmitoyl dipeptide-6 (KV), palmitoyl dipeptide-7 (KT), palmitoyl carnosine (AH), azelaoyl tripeptide-1 (GHK), palmitoyl-tripeptide-3 (GHR), myristoyl tripeptide-5 (KVK), palmitoyl tripeptide-1 (GHK), palmitoyl tripeptide-5 (KVK), palmitoyl tripeptide (RFK), myristoyl tripeptide-1 (GHK), palmitoyl tripeptide-4 (LGD), palmitoyl tripeptide-8 (HFR), palmitoyl tetrapeptide-7 (GQPR), myristoyl pentapeptide-17 (KLAKK), palmitoyl pentapeptide-4 (KTTKS), palmitoyl pentapeptide-17 (KLAKK), myristoyl hexapeptide-12 (VGVAPG) and palmitoyl hexapeptide-12 (VGVAPG).

4.   The metal layered hydroxide complex of claim 1, wherein the metal layered hydroxide complex is prepared using an outer ion-exchange process or a crossover interposition process.

5.   The metal layered hydroxide complex of claim 4, wherein the outer ion-exchange process enables formation of a structure configured such that an outermost surface of the metal layered hydroxide complex is surrounded by a surface modifier layer.

**6.** The metal layered hydroxide complex of claim 4, wherein the crossover interposition process enables formation of a structure configured such that the active ingredient and the surface modifier are provided in a mixture form inside and on an outermost surface of the metal layered hydroxide complex.

**7.** The metal layered hydroxide complex of claim 1, wherein the metal layered hydroxide complex has controlled releasability, in which a release rate of the active ingredient within 72 hr is 65% or less.

**8.** A method of preparing the metal layered hydroxide complex of claim 1, comprising:

preparing a precursor solution by adding an acidic solution dropwise to a precursor of a metal layered hydroxide complex and dissolving the precursor; and
preparing a metal layered hydroxide complex by mixing the precursor solution with an alcohol, deionized water, an active ingredient, and a surface modifier.

**9.** A method of preparing the metal layered hydroxide complex of claim 1, comprising:

preparing a precursor solution by adding an acidic solution dropwise to a precursor of a metal layered hydroxide complex and dissolving the precursor;
preparing a crystal seed containing an active ingredient by mixing the precursor solution with an alcohol, deionized water and an active ingredient; and
preparing a metal layered hydroxide complex by mixing the prepared crystal seed with a surface modifier and performing an ion exchange reaction, in which a pH in the ion exchange reaction is 5 to 10.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

Size Distribution by Intensity

|  | ① SMA1 0.05 : ascorbic acid 1 | ② SMA1 0.025 : ascorbic acid 1 | ③ SMA1 0.005 : ascorbic acid 1 | ④ SMA1 0.0025 : ascorbic acid 1 | ⑤ SMA1 0.0005 : ascorbic acid 1 | ⑥ SMA1 0 : ascorbic acid 1 |
|---|---|---|---|---|---|---|
| Z-average(d.nm) | 229.0 | 260.4 | 409.2 | 513.9 | 697.5 | 1018 |

FIG. 23

Size Distribution by Intensity

|  | ① SMA2 0.05 : ascorbic acid 1 | ② SMA2 0.025 : ascorbic acid 1 | ③ SMA2 0.005 : ascorbic acid 1 | ④ SMA2 0.0025 : ascorbic acid 1 | ⑤ SMA2 0.0005 : ascorbic acid 1 | ⑥ SMA2 0 : ascorbic acid 1 |
|---|---|---|---|---|---|---|
| Z-average(d.nm) | 271.8 | 425.9 | 602.5 | 680.4 | 906.6 | 1018 |

FIG. 24

48

Zeta Potential Distribution

| | ① SMA1 0.05 : ascorbic acid 1 | ② SMA1 0.025 : ascorbic acid 1 | ③ SMA1 0.005 : ascorbic acid 1 | ④ SMA1 0.0025 : ascorbic acid 1 | ⑤ SMA1 0.0005 : ascorbic acid 1 | ⑥ SMA1 0 : ascorbic acid 1 |
|---|---|---|---|---|---|---|
| Zeta potential (mV) | 39.5 | 28.7 | 20.4 | 17.3 | 16.9 | 13.6 |

FIG. 25

Zeta Potential Distribution

| | ① SMA2 0.05 : ascorbic acid 1 | ② SMA2 0.025 : ascorbic acid 1 | ③ SMA2 0.005 : ascorbic acid 1 | ④ SMA2 0.0025 : ascorbic acid 1 | ⑤ SMA2 0.0005 : ascorbic acid 1 | ⑥ SMA2 0 : ascorbic acid 1 |
|---|---|---|---|---|---|---|
| Zeta potential (mV) | 46.1 | 38.7 | 28.2 | 20.1 | 16.6 | 13.6 |

FIG. 26

Size Distribution by Intensity

| | ① SMA1 0.9995 : ascorbic acid 0.0005 | ② SMA1 0.995 : ascorbic acid 0.005 | ③ SMA1 0.5 : ascorbic acid 0.5 | ④ SMA1 0.005: ascorbic acid 0.995 | ⑤ SMA1 0.0005 : ascorbic acid 0.9995 | ⑥ SMA1 0 : ascorbic acid 1 |
|---|---|---|---|---|---|---|
| Z-average(d.nm) | 397.6 | 428.4 | 447.5 | 585.2 | 779.4 | 1018 |

FIG. 27

Size Distribution by Intensity

| | ① SMA2 0.9995 : ascorbic acid 0.0005 | ② SMA2 0.995 : ascorbic acid 0.005 | ③ SMA2 0.5 : ascorbic acid 0.5 | ④ SMA2 0.005: ascorbic acid 0.995 | ⑤ SMA2 0.0005 : ascorbic acid 0.9995 | ⑥ SMA2 0 : ascorbic acid 1 |
|---|---|---|---|---|---|---|
| Z-average(d.nm) | 231.7 | 444.1 | 513.9 | 625.8 | 798.7 | 1018 |

FIG. 28

Zeta Potential Distribution

|  | ① SMA1 0.9995 : ascorbic acid 0.0005 | ② SMA1 0.995 : ascorbic acid 0.005 | ③ SMA1 0.5 : ascorbic acid 0.5 | ④ SMA1 0.005: ascorbic acid 0.995 | ⑤ SMA1 0.0005 : ascorbic acid 0.9995 | ⑥ SMA1 0 : ascorbic acid 1 |
|---|---|---|---|---|---|---|
| Zeta potential (mV) | 28.4 | 27.6 | 25.2 | 21.0 | 14.2 | 13.6 |

FIG. 29

Zeta Potential Distribution

|  | ① SMA2 0.9995 : ascorbic acid 0.0005 | ② SMA2 0.995 : ascorbic acid 0.005 | ③ SMA2 0.5 : ascorbic acid 0.5 | ④ SMA2 0.005: ascorbic acid 0.995 | ⑤ SMA2 0.0005 : ascorbic acid 0.9995 | ⑥ SMA2 0 : ascorbic acid 1 |
|---|---|---|---|---|---|---|
| Zeta potential (mV) | 28.5 | 26.9 | 21.3 | 20.4 | 17.3 | 13.6 |

FIG. 30

FIG. 31

FIG. 32

FIG. 33

FIG. 34

FIG. 35

## INTERNATIONAL SEARCH REPORT

| International application No. |
|---|
| PCT/KR2020/005706 |

| A. CLASSIFICATION OF SUBJECT MATTER |
|---|
| *A61K 8/02(2006.01)i, A61K 8/27(2006.01)i, A61K 8/67(2006.01)i, A61K 8/64(2006.01)i, A61Q 19/00(2006.01)i, A61Q 19/10(2006.01)i* |
| According to International Patent Classification (IPC) or to both national classification and IPC |

**B. FIELDS SEARCHED**

| Minimum documentation searched (classification system followed by classification symbols) |
|---|
| A61K 8/02; A21D 2/00; A21D 2/08; A61K 31/07; A61K 47/48; A61K 8/67; A61K 8/97; A61Q 1/00; B01J 13/00; C07C 59/08; A61K 8/27; A61K 8/64; A61Q 19/00; A61Q 19/10 |

| Documentation searched other than minimum documentation to the extent that such documents are included in the fields searched |
|---|
| Korean utility models and applications for utility models: IPC as above<br>Japanese utility models and applications for utility models: IPC as above |

| Electronic data base consulted during the international search (name of data base and, where practicable, search terms used) |
|---|
| eKOMPASS (KIPO internal) & Keywords: active component, surface modifier, metal hydroxide complex, ion exchange |

**C. DOCUMENTS CONSIDERED TO BE RELEVANT**

| Category* | Citation of document, with indication, where appropriate, of the relevant passages | Relevant to claim No. |
|---|---|---|
| X | KR 10-2018-0021283 A (H&A PHARMACHEM CO., LTD.) 02 March 2018<br>See claims 1, 4, 6, 13, 33, 35. | 1-3,8 |
| Y | | 4-7,9 |
| Y | KR 10-0841700 B1 (NANOHYBRID CO., LTD.) 26 June 2008<br>See paragraphs [0059]-[0065]; example 24; figure 25. | 4-7,9 |
| A | KR 10-2006-0094745 A (NANOHYBRID CO., LTD.) 30 August 2006<br>See the entire document. | 1-9 |
| A | JP 2005-238194 A (NATIONAL INSTITUTE OF ADVANCED INDUSTRIAL &<br>TECHNOLOGY) 08 September 2005<br>See the entire document. | 1-9 |
| A | KR 10-2006-0132409 A (NANOHYBRID CO., LTD.) 21 December 2006<br>See the entire document. | 1-9 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 AUGUST 2020 (12.08.2020) | **12 AUGUST 2020 (12.08.2020)** |

| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br>Telephone No. |
|---|---|

Form PCT/ISA/210 (second sheet) (January 2015)

INTERNATIONAL SEARCH REPORT
Information on patent family members

International application No.

**PCT/KR2020/005706**

| Patent document cited in search report | Publication date | Patent family member | Publication date |
|---|---|---|---|
| KR 10-2018-0021283 A | 02/03/2018 | CN 109640927 A | 16/04/2019 |
| | | EP 3501484 A1 | 26/06/2019 |
| | | EP 3501484 A4 | 25/03/2020 |
| | | JP 2019-528287 A | 10/10/2019 |
| | | KR 10-1883719 B1 | 01/08/2018 |
| | | US 2019-0192396 A1 | 27/06/2019 |
| | | WO 2018-034548 A1 | 22/02/2018 |
| KR 10-0841700 B1 | 26/06/2008 | CN 1535131 A | 06/10/2004 |
| | | EP 1411882 A1 | 28/04/2004 |
| | | EP 1411882 A4 | 20/10/2004 |
| | | EP 1411882 B1 | 11/11/2009 |
| | | JP 2004-538296 A | 24/12/2004 |
| | | JP 4505591 B2 | 21/07/2010 |
| | | KR 10-0439299 B1 | 07/07/2004 |
| | | KR 10-2003-0015843 A | 25/02/2003 |
| | | KR 10-2004-0063878 A | 14/07/2004 |
| | | US 2004-0171735 A1 | 02/09/2004 |
| | | US 7288318 B2 | 30/10/2007 |
| | | WO 03-011233 A1 | 13/02/2003 |
| KR 10-2006-0094745 A | 30/08/2006 | US 2008-0153907 A1 | 26/06/2008 |
| | | WO 2006-091009 A1 | 31/08/2006 |
| JP 2005-238194 A | 08/09/2005 | JP 4572289 B2 | 04/11/2010 |
| KR 10-2006-0132409 A | 21/12/2006 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100439299
- KR 100841700
- KR 1020060132409